# EUROPEAN PATENT SPECIFICATION

(11) **EP 3 116 523 B1**
(45) Date of publication and mention of the grant of the patent: **17.10.2018**
(21) Application number: 15712232.6
(22) Date of filing: 10.03.2015
(51) Int. Cl.: A61K 38/10, A61L 31/04, A61K 9/00, A61L 26/00

(54) **SELF-ASSEMBLING PEPTIDES AS BRONCHIAL OBSTRUCTION AGENTS**
PEPTIDES AUTOASSÉMBLANTS EN TANT QU'AGENTS D' OBSTRUCTION BRONCHIQUE
SELBSTANORDNENDE PEPTIDE ALS WIRKSTOFFE FÜR BRONCHIENVERSTOPFUNG

(30) Priority: 10.03.2014 US 201461950529 P; 14.03.2014 US 201461953146 P
(43) Date of publication of application: 18.01.2017
(73) Proprietor: 3-D Matrix, Ltd., Tokyo 102-0083 (JP)
(72) Inventor: MEHTA, Manav, Brighton, MA 02135 (US); TSUKADA, Hisashi, Brooklline, MA 02446 (US); GIL, Eun, Seok, Acton, MA 01718 (US); GILBERT, Karl, Patrick, Danvers, MA 01923 (US); KOBAYASHI, Satoru, Kanagawa 253-0085 (JP)
(74) Representative: Barker Brettell LLP
(86) International application number: PCT/US2015/019740
(87) International publication number: WO 2015/138475

(56) References cited:
- EP-A1- 2 345 433
- WO-A2-03/096972
- WO-A2-2007/142757
- WO-A2-2013/030673
- C MOSER ET AL.: "Autologous fibrin sealant reduces the incidence of prolonged air leak and duration of the chest tube drainage after lung volume reduction surgery: a prospective randomized blinded study", JOURNAL OF THORACIC AND CARDIOVASCULAR SURGERY., vol. 136, no. 4, 2008, pages 843-849, XP25571241, MOSBY-YEAR BOOK, INC., ST. LOUIS, MO. ISSN: 0022-5223

### SEQUENCE LISTING

<110> 3-D MATRIX, LTD.
<120> BRONCHIAL OBSTRUCTION AGENT
<130> T2071-7011WO
<140>
   <141>
<150> 61/953,146
   <151> 2014-03-14
<150> 61/950,529
   <151> 2014-03-10
<160> 6
<170> PatentIn version 3.5
<210> 1
   <211> 16
   <212> PRT
   <213> Artificial Sequence
<220>
   <221> source
   <223> /note="Description of Artificial Sequence: Synthetic peptide"
<400> 1
   Arg Ala Asp Ala Arg Ala Asp Ala Arg Ala Asp Ala Arg Ala Asp Ala
   1 5 10 15
<210> 2
   <211> 13
   <212> PRT
   <213> Artificial Sequence
<220>
   <221> source
   <223> /note="Description of Artificial Sequence: Synthetic peptide"
<400> 2
   Ile Glu Ile Lys Ile Glu Ile Lys Ile Glu Ile Lys Ile
   1 5 10
<210> 3
   <211> 12
   <212> PRT
   <213> Artificial Sequence
<220>
   <221> source
   <223> /note="Description of Artificial Sequence: Synthetic peptide"
<400> 3
   Lys Leu Asp Leu Lys Leu Asp Leu Lys Leu Asp Leu
   1 5 10
<210> 4
   <211> 4
   <212> PRT
   <213> Artificial Sequence
<220>
   <221> source
   <223> /note="Description of Artificial Sequence: Synthetic peptide"
<400> 4
   Arg Ala Asp Ala
   1
<210> 5
   <211> 4
   <212> PRT
   <213> Artificial Sequence
<220>
   <221> source
   <223> /note="Description of Artificial Sequence: Synthetic peptide"
<400> 5
   Ile Glu Ile Lys
   1
<210> 6
   <211> 4
   <212> PRT
   <213> Artificial Sequence
<220>
   <221> source
   <223> /note="Description of Artificial Sequence: Synthetic peptide"
<400> 6
   Lys Leu Asp Leu
   1

## Description

### FIELD OF THE DISCLOSURE

This disclosure generally relates to materials and methods that may be used in medical, research, and industrial applications. More particularly, this disclosure relates to materials and methods that may be used as a bronchial obstruction agent, including materials and methods for use in long volume reduction surgery (LVRS) or other surgical procedures. WO-A2-2013030673 discloses the use of amphiphilic peptides in thoracic air leakage occlusion. EP-A1-2345433 discloses the use of amphiphilic peptides as tissue plug. J. Thor. Cardiovasc. Surgery 136(4), pages 843-849 (2008) discloses the use of autologous fibrin sealant in patients after lung volume reduction surgery.

### SUMMARY

In accordance with one or more embodiments, a method of providing a bronchial obstruction in a subject is disclosed. The method may comprise introducing a delivery device to a target area of the lung of the subject, positioning an end of the delivery device in the target area in which the bronchial obstruction is desired, administering through the delivery device a solution comprising a self-assembling peptide comprising between about 7 amino acids and 32 amino acids in an effective amount and in an effective concentration to the target area to form a hydrogel barrier under physiological conditions of the target area to provide the bronchial obstruction, and removing the delivery device from the target area.

In accordance with one or more embodiments, a kit for providing a bronchial obstruction in a subject is provided. The kit may comprise a self-assembling peptide comprising between about 7 amino acids and about 32 amino acids in an effective amount to form a hydrogel barrier under physiological conditions to provide the bronchial obstruction, and instructions for administering the self-assembling peptide to a target area of the lung of the subject.

In accordance with one or more embodiments, a composition comprising a self-assembling peptide comprising between about 7 amino acids and 32 amino acids in an effective amount and in an effective concentration for use in forming a hydrogel barrier under physiological conditions to provide a bronchial obstruction is disclosed.

In accordance with one or more embodiments, a method of facilitating providing a bronchial obstruction in a subject is disclosed. The method may comprise providing a solution comprising a self-assembling peptide comprising between about 7 amino acids to about 32 amino acids in an effective amount and in an effective concentration to form a hydrogel barrier in a target area of the lung under physiological conditions to provide the bronchial obstruction, and providing instructions for administering the solution to the target area of the lung through introduction of the solution through a delivery device positioned in the target area.

In accordance with one or more embodiments, a macroscopic scaffold consisting essentially of a plurality of self-assembling peptides is provided. Each of the self-assembling peptides comprises between about 7 amino acids and about 32 amino acids in an effective amount that is capable of being positioned within a target area of a lung to provide a bronchial obstruction.

### DESCRIPTION OF THE DRAWINGS

FIGS.1A-1B present data discussed in Example 1;
FIGS. 2-4 present data discussed in Example 3;
FIGS. 5A-5B present data discussed in Example 3;
FIGS. 6A-6B present data discussed in Example 4;
FIGS. 7A-7B present data discussed in Example 5;
FIGS. 8A-8B present data discussed in Example 5;
FIGS. 9A-9B present data discussed in Example 6;
FIGS. 10-12 present data discussed in Example 8;
FIGS. 13-14 present data discussed in Example 9;
FIGS. 15-16 present data discussed in Example 10;
FIGS. 17-18 present data discussed in Example 11;
FIG. 19 presents data discussed in Example 12;
FIG. 20 presents data discussed in Example 13;
FIG. 21 presents data discussed in Example 14;
FIG. 22 presents data discussed in Example 15;
FIGS. 23A-23B present data discussed in Example 16;
FIGS. 24A-24C present data discussed in Example 18;
FIGS. 25-28 present materials discussed in Example 19; FIG 28 discloses "IEIK" as SEQ ID NO: 5, and
FIG. 29 presents a schematic of a catheter/endotracheal tube combination discussed in Example 19.

### DETAILED DESCRIPTION

Chronic obstructive pulmonary disease (COPD) is currently the sixth leading cause of death worldwide and it is estimated to rise to the third leading cause by 2020. COPD may be characterized by slowly progressive development of airflow limitation that may be difficult to reverse. Treatments may include smoking cessation, medication, vaccination, respiratory rehabilitation, and oxygen inhalation therapy. These treatments may improve a subject's quality of life.

Other treatments may include lung volume reduction surgery (LVRS). This may be used in cases in which other treatments do not improve the health of the subject. LVRS may include lung resection procedures, airway bypass procedures, utilizing airway valves, utilizing lung volume reduction coils, bronchial thermal vapor ablation, or a biogel for bronchial obstruction. Lung resection is relatively invasive and may often cause postoperative complications. Use of a biogel may sometimes cause inflammation and there may be a risk of anaphylaxis.

The present disclosure relates to materials and methods for providing a bronchial obstruction. The materials and methods may comprise a self-assembling peptide hydrogel that may be used for providing a bronchial obstruction. In certain embodiments, the materials and methods of the present disclosure may provide or create a bronchial obstruction to, for example, reduce lung volume to improve the condition of a subject having COPD.

The materials and methods may provide a safer obstructive agent for lung volume reduction. It can be easily and repeatedly applied to the bronchi. The material may be introduced in place of or during lung volume reduction surgery. The material may be introduced to provide a bronchial obstruction in respiratory surgery. The material may be introduced to provide a bronchial obstruction in bronchoscopic surgery.

In accordance with one or more embodiments, a self-assembling peptide may provide, or create a bronchial obstruction. The material may prevent surgical procedures or reduce the frequency or extent of surgical procedures. The effect of introducing the self-assembling or self-organizing peptide may last for at least one month, and more typically may last for several months. This may be due to the sustainability of the material in the body of the subject. Accordingly, in some embodiments, it is contemplated that this treatment may require only infrequent injections or one injection. The bronchial obstruction may be partial or complete.

The self-assembling peptides of the present disclosure may include application, for example, administration of the self-assembling peptides to a predetermined or desired target area. The self-assembling peptide may be administered to a target area in the form of a peptide solution, hydrogel, membrane or other form. A target area may be a predetermined area of a subject that requires a particular treatment. In some embodiments, the target area may relate to a surgical site, such as a lung volume reduction site, or otherwise a portion of a lung, bronchi, thoracic cavity, or airway.

During self-assembly, the peptide may form nanofibers. The self-assembly may cause gelling of the peptide in solution. The gelling may provide or form a hydrogel. The peptide may form a beta-sheet spontaneously in the solution under neutral pH level. The peptide may form a beta-sheet spontaneously in the solution under physiological conditions and/or in the presence of a cation and/or anion.

The methods and materials of the present disclosure may be used during or after a surgical procedure. For example, the solution comprising the self-assembling peptide may be administered during or after a surgical procedure.

The methods of the present disclosure may comprise introducing a delivery device to a target area for bronchial obstruction in a subject. The method may provide positioning an end of the delivery device in the target area in which bronchial obstruction is desired.

The method of the present disclosure may also comprise administering the self-assembling peptides to a predetermined or desired target. The self-assembling peptide may be administered to a target area in the form of a peptide solution, hydrogel, membrane or other form. A target area may be a predetermined area of a subject that requires a particular treatment. In some embodiments, the target area may relate to a zone where bronchial obstruction is desired.

The administration may occur through the delivery device to the target area to form a hydrogel barrier. This may occur under physiological conditions of the target area to provide the bronchial obstruction.

The materials and methods may comprise providing a bronchial obstruction.

As used herein, the term "obstruction" is intended in include partial or complete occlusion or blockage of target area. For example, an upper, middle, and/or lower lobe of a lung may be obstructed so as to cause mechanical blockage therein. Penetration may be desired to cause the obstruction and to retain the obstruction. Generally, obstruction may be desired to treat a subject, such as a COPD patient. Treatment of a subject may include one or more of curing, alleviating, relieving or improving a subject with a disorder, beyond that expected in the absence of such treatment. The treatment may be a minimally invasive treatment, including minimally invasive application or administration of the solution comprising the self-assembling peptide.

As used herein, the term "subject" is intended to include human and non-human animals, for example, vertebrates, large animals, and primates. In certain embodiments, the subject is a mammalian subject, and in particular embodiments, the subject is a human subject. Although applications with humans are clearly foreseen, veterinary applications, for example, with non-human animals, are also envisaged herein. The term "non-human animals" of the invention includes all vertebrates, for example, non-mammals (such as birds, for example, chickens; amphibians; reptiles) and mammals, such as non-human primates, domesticated, and agriculturally useful animals, for example, sheep, dog, cat, cow, pig, rat, among others.

The treatment or obstruction may be partial or complete. The materials and methods may include administration, application, or injection of a self-assembling peptide, or a solution comprising a self-assembling peptide, or a composition comprising a self-assembling peptide, to a predetermined or desired target area.

The method of providing the bronchial obstruction may further comprise removing the delivery device from the target area. The method may further comprise visualizing a region comprising the target area prior to introducing the delivery device. Visualization of the region comprising the target area may occur subsequent to removing the delivery device from the target area. Monitoring of the target area may also occur during the procedure and subsequent to the procedure, for example, subsequent to removing the delivery device.

The method of providing the obstruction may further comprise preparing the solution comprising the self-assembling peptide. In some embodiments, the method may further comprise evaluating the subject to determine a need for providing a bronchial obstruction and preparing the solution based on the step of evaluating.

The term "self-assembling peptide" may refer to a peptide that may exhibit a beta-sheet structure in aqueous solution in the presence of specific conditions to induce the beta-sheet structure. These specific conditions may include adjusting the pH of a self-assembling peptide solution. The adjustment may be an increase or a decrease in the pH of the self-assembling peptide solution. The increase in pH may be an increase in pH to a physiological pH. The specific conditions may also include adding a cation, such as a monovalent cation or a divalent cation, to a self-assembling peptide solution. The specific conditions may also include adding an anion, such as a monovalent anion or a divalent anion, to a self-assembling peptide solution. The specific conditions may include conditions related to the site of a surgery or a target site for bronchial obstruction. The self-assembling peptides may be referred to as or be a part of a composition, peptide solution, peptide powder, hydrogel, or scaffold.

The term "self-assembling peptide" may refer to a peptide comprising a self-assembling motif. Self-assembling peptides are peptides that are capable of self-assembly into structures including but not limited to, macroscopic membranes or nanostructures.

The term "hydrogel" may refer to a material that is comprised of a polymer and a high percentage of water, for example, at least 90% water.

The self-assembling peptide may be an amphiphilic self-assembling peptide. By "amphiphilic" it is meant that the peptide comprises hydrophobic portions and hydrophilic portions. In some embodiments, an amphiphilic peptide may comprise, consist essentially of, or consist of alternating hydrophobic amino acids and hydrophilic amino acids. By alternating, it is meant to include a series of three or more amino acids that alternate between a hydrophobic amino acid and a hydrophilic amino acid, and it need not include each and every amino acid in the peptide sequence alternating between a hydrophobic and a hydrophilic amino acid. The self-assembling peptide, also referred to herein as "peptide," may be administered to the pre-determined or desired target area in the form of a self-assembling peptide solution, composition, hydrogel, membrane, scaffold or other form. The hydrogel may also be referred to as a membrane or scaffold throughout this disclosure. The pre-determined or desired target area may be at or near the location where a bronchial obstruction is desired.

The solution comprising a self-assembling peptide, also referred to as a self-assembling peptide solution, may be an aqueous self-assembling peptide solution. The self-assembling peptide may be administered, applied, or injected in a solution that is substantially cell-free, or free of cells. In certain embodiments, the self-assembling peptide may be administered, applied, or injected in a solution that is cell-free or free of cells.

The self-assembling peptide may also be administered, applied, or injected in a solution that is substantially drug-free or free of drugs. In certain embodiments, the self-assembling peptide may be administered, applied, or injected in a solution that is drug-free or free of drugs. In certain other embodiments, the self-assembling peptide may be administered, applied, or injected in a solution that is substantially cell-free and substantially drug-free. In still further certain other embodiments, the self-assembling peptide may be administered, applied, or injected in a solution that is cell-free and drug free.

The self-assembling peptide solution may comprise, consist of, or consist essentially of the self-assembling peptide. The self-assembling peptide may be in a modified or unmodified form. By modified, it is meant that the self-assembling peptide may have one or more domains that comprise one or more amino acids that, when provided in solution by itself, would not self-assemble. By unmodified, it is meant that the self-assembling peptide may not have any other domains other than those that provide for self-assembly of the peptide. That is, an unmodified peptide consists of alternating hydrophobic and hydrophilic amino acids that may self-assemble into a beta-sheet, and a macroscopic structure, such as a hydrogel.

Through administration of the solution comprising the self-assembling peptide, a hydrogel barrier may be formed. The hydrogel barrier may be formed in the target area to provide a bronchial obstruction. In some non-limiting embodiments, the treatment may be provided by blocking at least one lobe of a lung, at least partially. At least a portion of at least one lobe may be penetrated so as to cause an obstruction. This is accomplished through formation of the hydrogel barrier. Throughout this disclosure, reference to a hydrogel, may also refer to or be applicable to the hydrogel barrier.

In certain embodiments, it is desired to have the hydrogel barrier that may provide an adequate or desired blockage or obstruction at the target area. The hydrogel barrier may have specific properties to achieve the adequate or desired blockage or seal. For example, the hydrogel barrier may have one or more predetermined properties, for example, mechanical strength (storage modulus), rigidity, viscosity, gelation kinetics, ionic strength, or pH level. The properties may be adjusted or tailored based on the addition, to the self-assembling peptide or solution comprising the self-assembling peptide, of components disclosed herein in specific amounts and/or concentrations.

For example, related to providing a bronchial obstruction, it may be desired to provide a hydrogel barrier exhibiting appropriate mechanical properties, penetration and spreading within a lobe. Longer gelation time may be desired to promote penetration and spreading in some embodiments. It may also be desired to provide a hydrogel barrier that is quick to gel, i.e., the gelation kinetics are such that, upon administration, the hydrogel barrier is formed within a short amount of time to provide an obstruction.

Administration of a solution may comprise, consist of, or consist essentially of administration of a solution comprising, consisting of, or consisting essentially of a self-assembling peptide comprising, consisting of, or consisting essentially of at least about 7 amino acids. Administration of a solution may comprise, consist of, or consist essentially of administration of a solution comprising, consisting of, or consisting essentially of a self-assembling peptide comprising, consisting of, or consisting essentially of between about 7 amino acids and 32 amino acids. Other peptides that do not comprise, consist of, or consist essentially of at least about 7 amino acids may be contemplated by this disclosure.

The self-assembling peptide may comprise, consist of, or consist essentially of between about 7 to about 32 amino acids. In some embodiments, the self-assembling peptide may comprise, consist of, or consist essentially between about 12 and about 16 amino acids.

By alternating, it is meant to include a series of three or more amino acids that alternate between a hydrophobic amino acid and a hydrophilic amino acid, and it need not include each and every amino acid in the peptide sequence alternating between a hydrophobic and a hydrophilic amino acid.

The methods of providing a bronchial obstruction may comprise administering a self-assembling peptide to a target area. The peptide may be administered as a hydrogel or form a hydrogel upon administration. The methods of providing a bronchial obstruction may comprise administering a solution comprising a self-assembling peptide to a target area.

The term "administering," is intended to include, but is not limited to, applying, introducing or injecting the self-assembling peptide, in one or more of various forms including, but not limited to, by itself, by way of solution, such as an aqueous solution, or by way of a composition, hydrogel, or scaffold, with or without additional components.

The method may comprise introducing a delivery device to a target area of the subject. The method may comprise introducing a delivery device comprising at least one of a syringe, tube, pipette, catheter, catheter syringe, or other needle-based device to the target area of a subject. The self-assembling peptide may be administered by way of a syringe, tube, pipette, catheter, catheter syringe, or other needle-based device to the target area of a subject. The gauge of the syringe needle may be selected to provide an adequate flow of a composition, a solution, a hydrogel, or a liquid from the syringe to the target area. This may be based in some embodiments on at least one of the amount of self-assembling peptide in a composition, peptide solution, or a hydrogel being administered, the concentration of the peptide solution, in the composition, or the hydrogel, and the viscosity of the peptide solution, composition, or hydrogel. The delivery device may be a conventional device or designed to accomplish at least one of to reach a specific target area, achieve a specific dosing regime, deliver a specific target volume, amount, or concentration, and deliver accurately to a target area.

The method of providing a bronchial obstruction may comprise positioning an end of the delivery device in the target area. The target area may be an area as described herein, such as a portion of a surgical site, including but not limited to a portion of a lung. The self-assembling peptide may be administered by way of a delivery device to the target area in which providing a bronchial obstruction is desired. The self-assembling peptide may be administered in a solution by way of the delivery device to the target area. In some embodiments, the administration may occur directly at the target site, in that the delivery device is positioned at the target area to provide the solution comprising the self-assembling peptide to, for example, directly to, the target area. A predetermined volume of the target area may be filled with the solution comprising the self-assembling peptide. Administering the solution may comprise injecting the solution into the target area. In some embodiments, a delivery device is inserted through a trachea and primary bronchus into a distal end of a target lobe. The delivery device may be retracted a predetermined distance after injection of a first volume of hydrogel. The process may be repeated iteratively between injection and retraction until a target volume is filled to create a bronchial obstruction. This may be referred to as a catheter retraction process. In some embodiments, the peptide hydrogel may substantially penetrate both major and minor lumens of related bronchi.

The use of a delivery device may provide a more selective administration of the peptide to provide for a more accurate delivery to the target area. Selective administration of the peptide may allow for enhanced and more targeted delivery of the peptide solution, composition, or hydrogel such that is successful and positioned in the desired location in an accurate manner. The selective administration may provide enhanced, targeted delivery that markedly improves the positioning and effectiveness of the treatment over use of another delivery device. Delivery devices that may be used in the systems, methods, and kits of the disclosure may include a syringe, tube, needle, pipette, syringe catheter, other needle-based device, or catheter.

Use of a delivery device, such as a catheter, may include use of accompanying devices, such as a guidewire used to guide the catheter into position, or an endoscope that may allow proper placement of a catheter or other device and visualization of the target area, and/or the path to the target area. The endoscope may be a tube that may comprise at least one of a light and a camera or other visualization device to allow images of the subject's body to be viewed. The guidewire or endoscope may be introduced into the subject, for example, by way of an incision in the skin. The endoscope may be introduced to the target area prior to introducing the delivery device to the target area.

In some embodiments, an endotracheal tube combined with a catheter may be used to prevent backflow of the hydrogel out of the lumen during injection. A balloon-type cathether configuration may also beneficially increase pressure for enhanced perfusion of gel, such as into smaller bronchi.

The use of the delivery device, such as a syringe, tube, needle, pipette, syringe catheter, other needle-based device, catheter, or endoscope may require determining the diameter or size of the opening in which there is a target area, such that at least a portion of the syringe, tube, needle, pipette, syringe catheter, other needle-type device, catheter, or endoscope may enter the opening to administer the peptide, peptide solution, composition, or hydrogel to the target area.

In certain embodiments, the hydrogel may be formed *in vitro* and administered to the desired location *in vivo.* In certain examples, this location may be the target area. In other examples, this location may be upstream, downstream of the area, or substantially near the area. It may be desired to allow a migration of the hydrogel to the area in which it is desired to gel. Alternatively, another procedure may position the hydrogel in the area in which it is desired. The desired location or target area may be at least a portion of an area in which it is desired to form a bronchial obstruction in a subject.

In certain aspects of the disclosure, the hydrogel may be formed *in vivo.* A solution comprising the self-assembling peptide, such as an aqueous solution, may be inserted to an *in vivo* location or area of a subject to form a bronchial obstruction in a subject. In certain examples, the hydrogel may be formed *in vivo* at one location, and allowed to migrate to the area in which it is desired to promote or provide a bronchial obstruction, for example, a lung volume reduction in a COPD patient. Alternatively, another procedure may place the hydrogel in the area in which it is desired to promote or provide a bronchial obstruction. The peptides of the present disclosure may be in the form of a powder, a solution, a gel, or the like. Since the self-assembling peptide gels in response to changes in solution pH and salt concentration, it can be distributed as a liquid that gels upon contact with a subject during application or administration.

In certain environments, the peptide solution may be a weak hydrogel and, as a result, it may be administered by way of a delivery device as described herein.

In accordance with some embodiments, the self-assembling peptides may be amphiphilic, alternating between hydrophobic amino acids and hydrophilic amino acids.

In accordance with one or more embodiments, a subject may be evaluated to determine a need to provide a bronchial obstruction in a subject. Once the evaluation has been completed, a peptide solution to administer to the subject may be prepared based on the evaluating step. In other embodiments, a peptide solution may be prepared without the step of evaluating.

In some embodiments, a biologically active agent may be used with the materials and methods of the present disclosure. A biologically active agent may comprise a compound, including a peptide, DNA sequence, chemical compound, or inorganic or organic compound that may impart some activity, regulation, modulation, or adjustment of a condition or other activity in a subject or in a laboratory setting. The biologically active agent may interact with another component to provide such activity. The biologically active agent may be referred to as a drug in accordance with some embodiments herein. In certain embodiments, one or more biologically active agents may be gradually released to the outside of the peptide system. For example, the one or more biologically active agents may be gradually released from the hydrogel. Both *in vitro* and *in vivo* testing has demonstrated this gradual release of a biologically active agent. The biologically active agent may be added to the self-assembling peptide solution or composition prior to administering to a subject, or may be administered in conjunction with the self-assembling peptide or separately from the self-assembling peptide to the subject. The one or more biologically active agents may be encapsulated within the system, for example, they may be encapsulated in the hydrogel, solution, composition, or nanofibers.

This disclosure relates to aqueous solutions, hydrogels, scaffolds, compositions and membranes comprising self-assembling peptides, sometimes referred to as self-assembling oligopeptides. The self-assembling peptides may exhibit a beta-sheet structure in aqueous solution in the presence of physiological pH and/or cations and/or anions, such as a monovalent cation and/or monovalent anion, or other conditions applicable to a surgical site or at or near the site of a desired bronchial obstruction. The peptides may be amphiphilic and alternate between a hydrophobic amino acid and a hydrophilic amino acid. In certain embodiments, the peptide may comprise a first portion that may be amphiphilic, alternating between a hydrophobic amino acid and a hydrophilic amino acid, and another portion or region that is not amphiphilic.

The peptides may be generally stable in aqueous solutions and self-assemble into large, macroscopic structures, scaffolds, or matrices when exposed to selected conditions. The conditions may be physiological conditions, neutral pH, selected concentrations of salts, buffer solutions, or physiological levels of salt. Once the hydrogel is formed it may not decompose, or may decompose or biodegrade after a period of time. The rate of decomposition may be based at least in part on at least one of the amino acid sequence and conditions of its surroundings. The rate of decomposition may be related to the rate of healing or growth at the target site, so as to provide a suitable bronchial obstruction.

By "macroscopic" it is meant as having dimensions large enough to be visible under magnification of 10-fold or less. In preferred embodiments, a macroscopic structure is visible to the naked eye. A macroscopic structure may be transparent and may be two-dimensional, or three-dimensional. Typically each dimension is at least 10 µm, in size. In certain embodiments, at least two dimensions are at least 100 µm, or at least 1000 µm in size. Frequently at least two dimensions are at least 1-10 mm in size, 10-100 mm in size, or more.

In certain embodiments, the size of the filaments may be about 10 nanometers (nm) to about 20 nm. The interfilament distance may be about 50 nm to about 80 nm.

The macroscopic structure may be a macroscopic scaffold. The macroscopic scaffold may consist essentially of a plurality of self-assembling peptides. Each of the self-assembling peptides may comprise, consist essentially of, or consist of between about 7 amino acids and about 32 amino acids in an effective amount that is capable of being positioned within a target area of a pulmonary system to provide a bronchial obstruction. The self-assembling peptides of the scaffold may comprise between about 12 to about16 amino acids. The self-assembling peptides of the scaffold may comprise between about 12 to about 16 amino acids that alternate between a hydrophobic amino acid and a hydrophilic amino acid. The self-assembling peptide may comprise, consist essentially of, or consist of (RADA)₄ (SEQ ID NO: 1), (IEIK)₃I (SEQ ID NO: 2), (KLDL)₃ (SEQ ID NO: 3).

"Physiological conditions" may occur in nature for a particular organism, cell system, or subject which may be in contrast to artificial laboratory conditions. The conditions may comprise one or more properties such as one or more particular properties or one or more ranges of properties. For example, the physiological conditions may include a temperature or range of temperatures, a pH or range of pH's, a pressure or range of pressures, and one or more concentrations of particular compounds, salts, and other components. The salts may comprise one or more of monovalent anions, monovalent cations, divalent anions, or monovalent cations.

In some examples, the physiological conditions may include a temperature in a range of about 20 to about 40 degrees Celsius. In some examples, the atmospheric pressure may be about 1 atm. The pH may be in the range of a neutral pH. For example, the pH may be in a range of about 6 to about 8. The physiological conditions may include cations and/or anions such as monovalent metal cations and/or monovalent anions that may induce membrane or hydrogel formation. These may include sodium chloride (NaCl). The physiological conditions may also include a glucose concentration, sucrose concentration, or other sugar concentration, of between about 1 mM and about 20 mM. The self-assembling peptide solution may comprise glucose, sucrose, or other sugar, or a sugar or sugar solution may be added to the self-assembling peptide solution.

In certain embodiments, the self-assembling peptides may be peptides of at least about 7 amino acids. In certain further embodiments, the self-assembling peptides may be peptides of at least about 7 amino acids to about 32 amino acids. In certain further embodiments, the self-assembling peptides may be peptides of between about 7 to about 17 amino acids. In certain other examples, the self-assembling peptides may be peptides of at least 8 amino acids, at least about 12 amino acids, or at least about 16 amino acids.

Both homogeneous and heterogeneous mixtures of peptides characterized by the above-mentioned properties may form stable macroscopic membranes, filaments, and hydrogels. Peptides which are self-complementary and self-compatible may form membranes, filaments, and hydrogels in a homogeneous mixture. Heterogeneous peptides, including those which cannot form membranes, filaments, and hydrogels in homogeneous solutions, which are complementary and/or structurally compatible with each other may also self-assemble into macroscopic membranes, filaments, and hydrogels.

The membranes, filaments, and hydrogels may be non-cytotoxic. The hydrogels of the present disclosure may be digested and metabolized in a subject. The hydrogels may be biodegraded in 30 days or less. They have a simple composition, are permeable, and are easy and relatively inexpensive to produce in large quantities. The membranes and filaments, hydrogels or scaffolds may also be produced and stored in a sterile condition. The optimal lengths for membrane formation may vary with at least one of the amino acid composition, solution conditions, and conditions at the target area.

The amino acids of the self-assembling or amphiphilic peptides may be selected from d-amino acids, 1-amino acids, or combinations thereof. The hydrophobic amino acids may include Ala, Val, Ile, Met, Phe, Tyr, Trp, Ser, Thr and Gly. The hydrophilic amino acids may be basic amino acids, for example, Lys, Arg, His, Orn; acidic amino acids, for example, Glu, Asp; or amino acids which form hydrogen bonds, for example, Asn, Gln. Acidic and basic amino acids may be clustered on a peptide. The carboxyl and amino groups of the terminal residues may be protected or not protected. Membranes or hydrogels may be formed in a homogeneous mixture of self-complementary and self-compatible peptides or in a heterogeneous mixture of peptides which are complementary and structurally compatible to each other. Peptides fitting the above criteria may self-assemble into macroscopic membranes under suitable conditions, described herein.

In certain embodiments, about 8 to about 32 residues may be used in the self-assembling peptides, while in other embodiments self-assembling peptides may have about 7 to about 17 residues. The peptides may have a length of about 5 nm.

The peptides of the present disclosure may comprise, consist essentially of, or consist of peptides having the repeating sequence of arginine, alanine, aspartic acid and alanine (Arg-Ala-Asp-Ala (SEQ ID NO: 4) (RADA (SEQ ID NO: 4))).

Other peptide sequences may be represented by self-assembling peptides comprising, consisting essentially of, or consisting of the repeating sequence of isoleucine, glutamic acid, isoleucine and lysine (Ile-Glu-Ile-Lys (SEQ ID NO: 5) (IEIK (SEQ ID NO: 5))). Other peptide sequences may be represented by self-assembling peptides comprising, consisting essentially of, or consisting of the repeating sequence of lysine, leucine, aspartic acid, and leucine (Lys-Leu-Asp-Leu (SEQ ID NO: 6) (KLDL (SEQ ID NO: 6))). As specific examples of self-assembling peptides according to the invention there may be a self-assembling peptide referred to as "RADA16" having the sequence Arg-Ala-Asp-Ala-Arg-Ala-Asp-Ala- Arg-Ala-Asp-Ala-Arg-Ala-Asp-Ala (SEQ ID NO: 1) ((RADA)₄ (SEQ ID NO: 1)) (also referred to as "Puramatrix" throughout the disclosure), a self-assembling peptide referred to as "IEIK13" having the sequence Ile-Glu-Ile-Lys-Ile-Glu-Ile-Lys-Ile-Glu-Ile-Lys-Ile (SEQ ID NO: 2) ((IEIK)₃I (SEQ ID NO: 2)), or a self-assembling peptide referred to as "KLDL12" (which may also be referred to as "KLD12" throughout this disclosure) having the sequence Lys-Leu-Asp-Leu-Lys-Leu-Asp-Leu-Lys-Leu-Asp-Leu (SEQ ID NO: 3) ((KLDL)₃ (SEQ ID NO: 3)).

Each of the peptide sequences disclosed herein may provide for peptides comprising, consisting essentially of, and consisting of the amino acid sequences recited.

The present disclosure provides materials, methods, and kits for solutions, hydrogels, compositions, and scaffolds comprising, consisting essentially of, or consisting of the peptides recited herein.

A 1 weight per volume (w/v) percent aqueous (water) solution and a 2.5 w/v percent of (RADA)₄ (SEQ ID NO: 1) is available as the product PuraMatrix™ peptide hydrogel by 3-D Matrix Co., Ltd.

The self-assembly of the peptides may be attributable to hydrogen bonding and hydrophobic bonding between the peptide molecules by the amino acids composing the peptides.

The self-assembling peptides of the present disclosure may have a nanofiber diameter in a range of about 10 nm to about 20 nm and an average pore size is in a range of about 5 nm to about 200 nm. In certain embodiments, the nanofiber diameter, the pore size, and the nanofiber density may be controlled by at least one of the concentration of peptide solution used and the amount of peptide solution used, such as the volume of peptide solution.

As such, at least one of a specific concentration of peptide in solution and a specific amount of peptide solution to provide at least one of a desired nanofiber diameter, pore size, and density to adequately provide for forming a bronchial obstruction may be selected. The specific concentration and specific amount of peptide solution may be referred to as an "effective concentration" and an "effective amount."

As used herein, an amount of a peptide, peptide solution or hydrogel effective to provide a bronchial obstruction in a subject, an "effective amount" or a "therapeutically effective amount" refers to an amount of the peptide, peptide solution, composition, or hydrogel, which is effective, upon single or multiple administration (application or injection) to a subject, in treating, or in curing, alleviating, relieving or improving a subject with a disorder beyond that expected in the absence of such treatment. This may include a particular concentration or range of concentrations of peptide in the peptide solution, composition, or hydrogel and additionally, or in the alternative, a particular volume or range of volumes of the peptide solution, composition, or hydrogel. The method of facilitating may comprise providing instructions to prepare at least one of the effective amount and the effective concentration.

The dosage, for example, volume or concentration, administered (for example, applied or injected) may vary depending upon the form of the peptide (for example, in a peptide solution, hydrogel, or in a dried form, such as a lyophilized form) and the route of administration utilized. The exact formulation, route of administration, volume, and concentration can be chosen in view of the subject's condition and in view of the particular target area or location that the peptide solution, hydrogel, or other form of peptide will be administered. Lower or higher doses than those recited herein may be used or required. Specific dosage and treatment regimens for any particular subject may depend upon a variety of factors, which may include the specific peptide or peptides employed, the dimension of the area that is being treated, the desired thickness of the resulting hydrogel that may be positioned in the desired target area, and the length of time of treatment. Other factors that may affect the specific dosage and treatment regimens include age, body weight, general health status, sex, time of administration, rate of degradation, the severity and course of the disease, condition or symptoms, and the judgment of the treating physician. In certain embodiments, the peptide solution may be administered in a single dose. In other embodiments, the peptide solution may be administered in more than one dose, or multiple doses. The peptide solution may be administered in at least two doses.

An effective amount and an effective concentration of the peptide solution may be selected to at least partially obstruct a bronchial region, for example to promote or provide a lung volume reduction in a subject. In some embodiments, at least one of the effective amount and the effective concentration may be based in part on a dimension or diameter of the target area, such as at least one lung lobe. In other embodiments, at least one of the effective amount and the effective concentration is based in part on the flow rate of one or more fluids at or near the target area. In still other embodiments, at least one of the effective amount and the effective concentration may be based in part on a dimension or diameter of the target area of the bronchial site or the site of a surgery.

In yet other embodiments, at least one of the effective amount and the effective concentration may be based in part on at least one of a dimension or diameter of the target area, and the flow rate of one or more fluids at or near the target area, and a dimension or diameter of a target area for bronchial obstruction, for example, one or more lung lobes.

The effective amount may include volumes of from about 0.1 milliliters (mL) to about 100 mL of a peptide solution. The effective amount may include volumes of from about 0.1 mL to about 10 mL of a peptide solution. The effective amount may include volumes of from about 0.1 to about 5 mL. In certain embodiments, the effective amount may be about 0.4 mL. In certain embodiments, the effective amount may be about 0.5 mL. In other embodiments, the effective amount may be about 1.0 mL. In yet other embodiments, the effective amount may be about 1.5 mL. In still yet other embodiments, the effective amount may be about 2.0 mL. In some other embodiments, the effective amount may be about 3.0 mL.

In certain embodiments, the effective amount may be approximately 0.1 mL per 1 cm² to approximately 5 mL per 1 cm² of target area. The effective amount may be about 0.1 mL per 1 cm² to about 1.5 mL per 1 cm². In certain embodiments, the effective amount may be approximately 1 mL per 1 cm² of target area. This effective amount may be used related to a concentration, such as a 1.5 weight per volume percent or a 2.5 weight per volume percent of a peptide solution of the present disclosure.

The effective concentration may be, as described herein, an amount that may form the bronchial obstruction. Various properties at or near the target site may contribute to the selection or determination of the effective concentration including at least one of a dimension or diameter of the target area, and the flow rate of one or more fluids at or near the target area.

The effective concentration may include peptide concentrations in the solution in a range of about 0.1 weight per volume (w/v) percent to about 10 w/v percent. The effective concentration may include peptide concentrations in the solution in a range of about 0.1 w/v percent to about 3.5 w/v percent. In certain embodiments, the effective concentration may be about 1 w/v percent. In certain other embodiments, the effective concentration may be about 1.5 w/v percent. In other embodiments, the effective concentration may be about 2.5 w/v percent. In yet other embodiments, the effective concentration may be about 3.0 w/v percent.

In certain embodiments, a peptide solution having a higher concentration of peptide may provide for a more effective hydrogel that has the ability to stay in place and provide effective treatment. For purposes of delivering the peptide solution, higher concentrations of peptide solutions may become too viscous to allow for effective and selective administration of the solution. It is possible that if a high enough concentration is not selected, the hydrogel may not be effective in the target area for the desired period of time.

The effective concentration may be selected to provide for a solution that may be administered by injection or other means using a particular diameter or gauge catheter or needle.

Methods of the disclosure contemplate single as well as multiple administrations of a therapeutically effective amount of the peptides, compositions, peptide solutions, membranes, filaments, and hydrogels as described herein. Peptides as described herein may be administered at regular intervals, depending on the nature, severity and extent of the subject's condition. In some embodiments, a peptide, composition, peptide solution, membrane, filament, or hydrogel may be administered in a single administration. In some embodiments, a peptide, composition, peptide solution, or hydrogel described herein is administered in multiple administrations. In some embodiments, a therapeutically effective amount of a peptide, composition, peptide solution, membrane, filament, or hydrogel may be administered periodically at regular intervals. The regular intervals selected may be based on any one or more of the initial peptide concentration of the solution administered, the amount administered, and the degradation rate of the hydrogel formed. For example, after an initial administration, a follow-on administration may occur after, for example, 30 seconds, 1 minute, two minutes, 5 minutes, 10 minutes, 1 day, 2 days, 5 days, one week, two weeks, four weeks, six weeks, or eight weeks. The follow-on administration may comprise administration of a solution having the same concentration of peptide and volume as the initial administration, or may comprise administration of a solution of lesser or great concentration of peptide and volume. The selection of the appropriate follow-on administration of peptide solution may be based on imaging the target area and the area surrounding the target area and ascertaining the needs based on the condition of the subject. The pre-determined intervals may be the same for each follow-on administration, or they may be different. In some embodiments, a peptide, peptide solution, or hydrogel may be administered chronically at pre-determined intervals to maintain at least a partial bronchial obstruction in a subject over the life of the subject. The pre-determined intervals may be the same for each follow-on administration, or they may be different. This may be dependent on whether the hydrogel formed from the previous administration is partially or totally disrupted or degraded. The follow-on administration may comprise administration of a solution having the same concentration of peptide and volume as the initial administration, or may comprise administration of a solution of lesser or great concentration of peptide and volume. The selection of the appropriate follow-on administration of peptide solution may be based on imaging or visualizing the target area and the area surrounding the target area and ascertaining the needs based on the condition of the subject.

Administration of the self-assembling peptide may comprise applying a solution comprising the self-assembling peptide through or into the target area. For example, a delivery device may be positioned within the bronchial area so as to administer, for example, inject the solution into the bronchial area.

These administration procedures may be accomplished through appropriate positioning of the delivery device. As discussed above, the delivery device may be a syringe. The syringe may have a particular gauge in order to allow proper flow of the solution onto or into the target area in order to achieve bronchial obstruction.

Further procedures regarding treatment may comprise administering a salt solution to the target area prior, during, or subsequent to applying the solution comprising the self-assembling peptide. This may provide superior obstruction due to increase in the mechanical strength of the resulting hydrogel barrier, for example, increased storage modulus of the resulting hydrogel barrier as compared to a hydrogel barrier that does not include treatment with a salt solution.

The self-assembling peptides of the present disclosure, such as RADA16, may be peptide sequences that lack a distinct physiologically or biologically active motif or sequence, and therefore may not impair intrinsic cell function. Physiologically active motifs may control numerous intracellular phenomena such as transcription, and the presence of physiologically active motifs may lead to phosphorylation of intracytoplasmic or cell surface proteins by enzymes that recognize the motifs. When a physiologically active motif is present in a peptide, transcription of proteins with various functions may be activated or suppressed. The self-assembling peptides, of the present disclosure may lack such physiologically active motifs and therefore do not carry this risk.

A sugar may be added to the self-assembling peptide solution to improve the osmotic pressure of the solution from hypotonicity to isotonicity without reducing the bronchial obstruction, thereby allowing the biological safety to be increased. In certain examples, the sugar may be sucrose or glucose.

The optimal lengths for membrane formation may vary with the amino acid composition. A stabilization factor contemplated by the peptides of the present disclosure is that complementary peptides maintain a constant distance between the peptide backbones. Peptides which can maintain a constant distance upon pairing are referred to herein as structurally compatible. The interpeptide distance can be calculated for each ionized or hydrogen bonding pair by taking the sum of the number of unbranched atoms on the side-chains of each amino acid in the pair. For example, lysine has 5 and glutamic acid has 4 unbranched atoms on its side-chains, respectively. The peptides can be chemically synthesized or they can be purified from natural and recombinant sources. Using chemically synthesized peptides may allow the peptide solutions to be deficient in unidentified components such as unidentified components derived from the extracellular matrix of another animal or microorganism. This property therefore may eliminate concerns of infection, including risk of viral infection compared to conventional tissue-derived biomaterials. This may eliminate concerns of infection including infections such as bovine spongiform encephalopathy (BSE), making the peptide highly safe for treatment of pulmonary leakage.

The initial concentration of the peptide may be a factor in the size and thickness of the membrane, hydrogel, or scaffold formed. In general, the higher the peptide concentration, the higher the extent of membrane or hydrogel formation. Hydrogels, or scaffolds formed at higher initial peptide concentrations (about 10 mg/ml) (about 1.0 w/v percent) may be thicker and thus, likely to be stronger.

Formation of the, membranes, hydrogels, compositions, or scaffolds may be very fast, on the order of a few seconds or a few minutes. The formation of the membranes or hydrogels may be irreversible. In certain embodiments, the formation may be reversible. The hydrogel may form instantaneously upon administration to a target area. The formation of the hydrogel may occur within about one to two minutes of administration. In other examples, the formation of the hydrogel may occur within about three to four minutes of administration. In certain embodiments the time it takes to form the hydrogel may be based at least in part on one or more of the concentration of the peptide solution, the volume of peptide solution applied, and the conditions at the area of application or injection (for example, the concentration of monovalent metal cations and/or anions at the area of application, the pH of the area, and the presence of one or more fluids at or near the area, additional components added to the solution prior to or subsequent to administration to the target area). The process may be unaffected by pH of less than or equal to 12, and by temperature. The membranes or hydrogels may form at temperatures in the range of 1 to 99 degrees Celsius.

The hydrogels may remain in position at the target area for a period of time sufficient to provide a desired effect using the methods and kits of the present disclosure. The desired effect using the materials, compositions, methods and kits of the present disclosure may be to treat areas or to assist in healing of areas in which a surgical procedure at or near the site of a surgery was performed or the site of a desired bronchial obstruction, such as but not limited to a lung volume reduction. For example, the desired effect using the materials, compositions, methods and kits of the present disclosure may be to assist in a pulmonary surgery or procedure to provide a bronchial obstruction.

The materials and methods of the present disclosure, including use of a solution, hydrogel, composition, or membrane comprising a self-assembling peptide as described herein to provide a bronchial obstruction, are provided in order to produce a hydrogel barrier in a target area. A property of the hydrogel barrier that may determine the adequacy of success of the treatment or procedure is burst pressure, or burst pressure tolerance. Burst pressure may refer to the pressure at which the hydrogel barrier will fail. For example, this may be the pressure at which the hydrogel barrier no longer operates as desired to provide a suitable treatment to the target area. The burst pressure may be the pressure at which the blockage or occlusion or obstruction provided by the hydrogel barrier allows air to pass through.

In some embodiments, it may be desirable to provide, through use of the materials and methods of the disclosure, a burst pressure that is similar to or higher than that which is achieved with normal tissue (for example, undamaged tissue). It may be desirable to provide, through use of the materials and methods of the disclosure, a burst pressure that is similar to the pressures exhibited through ordinary or average lung function. There is an effect of gelation time, post-application. For normal, healthy tissue, a burst pressure of about 20 to about 30 cmH₂O may be generally observed.

The period of time that the membranes or hydrogels may remain at the desired area may be for about 10 minutes. In certain examples, it may remain at the desired area for about 35 minutes. In certain further examples, it may remain at the desired area for one or more days, up to one or more weeks. In other examples, it may remain at the desired area for up to 30 days, or more. It may remain at the desired area indefinitely. In other examples, it may remain at the desired area for a longer period of time, until it is naturally degraded or intentionally removed. If the hydrogel naturally degrades over a period of time, subsequent application or injection of the hydrogel to the same or different location may be performed.

In certain embodiments, the self-assembling peptide may be prepared with one or more components that may provide for enhanced effectiveness of the self-assembling peptide or may provide another action, treatment, therapy, or otherwise interact with one or more components of the subject. The one or more other components may provide for higher mechanical strength, as measured by storage modulus, G' and improved gelation kinetics, for example, faster gelation into a hydrogel or hydrogel barrier.

For example, the pH of the self-assembling peptide, for example, in the form of a self-assembling peptide solution or composition, may be adjusted. The pH of the self-assembling peptide, in the form of a self-assembling peptide solution or composition, may be increased or decreased. This may be done by adjusting the pH of the self-assembling peptide solution, by way of addition of a pH adjuster. The pH adjuster may be, for example, salts, a salt solution or buffer solution. The pH adjuster may be selected based on the amino acid sequence of the self-assembling peptide, the type of salt or salts, the concentration of the one or more salts, and the pH of the pH adjuster. In certain embodiments, the pH of the solution comprising the self-assembling peptide is between about 2.5 to about 4.0.

The solutions and compositions comprising self-assembling peptides that are provided by this disclosure may be prepared with additional components, for example, one or more salts. Preparation of the solution may comprise adding the self-assembling peptide, for example, in the form of a peptide powder or a peptide solution, to a salt solution. In other embodiments, the preparation of the solution may comprise adding a salt or a salt solution to a self-assembling peptide, in the form of a peptide powder or a peptide solution. In other embodiments, the preparation of the solution comprising the self-assembling peptide comprises adding water to a peptide powder of the self-assembling peptide to provide an aqueous peptide solution. The water may be deionized water or any purified water suitable for peptide solution preparation. The water may be medical device acceptable grade or pharmaceutically acceptable grade. The peptide powder and water may be optionally mixed. A salt or salt solution may then be added to the aqueous peptide solution. The salt or salt solution and the aqueous peptide solution may then be mixed.

Salt solutions may be provided to use in the solution comprising the self-assembling peptide, to add to the solution comprising the self-assembling peptide, or to add to the hydrogel or composition comprising the self-assembling peptide. The salt solutions may be provided with specific anions and cations, and at specific concentrations in order to impart a desired property to the solution comprising the self-assembling peptide, or the resulting hydrogel, or hydrogel barrier. For example the salt solution may be provided to have a mechanical strength (storage modulus), rigidity, viscosity, gelation kinetics, ionic strength, pH, or burst pressure (burst pressure tolerance).

Salt solutions may comprise monovalent and/or divalent cations and/or anions. The salt solution may comprise at least one cation selected from the group consisting of ammonium, iron, magnesium, potassium, pyrimidium, quaternary ammonium, sodium, and calcium. The salt solution may comprise at least one anion selected from the group consisting of chloride, sulfate, acetate, carbonate, citrate, cyanide, fluoride, nitrate, nitrite, and phosphate.

In some embodiments, the salt solution comprises at least one of calcium chloride, sodium chloride, and potassium chloride.

In certain embodiments, the solution comprising the self-assembling peptide may comprise (RADA)₄ (SEQ ID NO: 1) at a concentration of about 0.5 w/v percent. This solution may further comprise a calcium chloride concentration of about 0.125 M. This solution may further provide for a storage modulus of about 25 Pa.

In certain embodiments, the solution comprising the self-assembling peptide may comprise (RADA)₄ (SEQ ID NO: 1) at a concentration of about 0.5 w/v percent. This solution may further comprise a calcium chloride concentration of about 0.250 M. This solution may further provide for a storage modulus of about 44 Pa.

In certain embodiments, the solution comprising the self-assembling peptide may comprise (RADA)₄ (SEQ ID NO: 1) at a concentration of about 0.5 w/v percent. This solution may further comprise a calcium chloride concentration of about 0.500 M. This solution may further provide for a storage modulus of about 52 Pa.

In certain embodiments, the solution comprising the self-assembling peptide may comprise (RADA)₄ (SEQ ID NO: 1) at a concentration of about 2.5 w/v percent. This solution may further comprise a calcium chloride concentration of about 0.125 M. This solution may further provide for a storage modulus of about 600 Pa.

In embodiments, the solution comprising the self-assembling peptide may have a concentration of salt of between about 0.005 M and about 1 M. In certain embodiments, the solution comprising the self-assembling peptide may have a concentration of salt of between about 0.125 M and about 0.500 M. In certain embodiments, the solution comprising the self-assembling peptide may have a concentration of salt of between of about 0.25 M.

In embodiments, the solution comprising the self-assembling peptide may comprise or may have added to it an isotonic solution. The isotonic solution may be relative to a subject, for example bodily fluids of the subject or the local physiological conditions at the target area. The isotonic solution may comprise at least one of sodium chloride, potassium chloride calcium chloride and water. The solution may contain hydrochloric acid or sodium hydroxide, which may be used for pH adjustment. To prepare this solution 8.6g NaCl, 0.3g KCl, 0.33g CaCl₂ may be dissolved in one litre of distilled water. The pH of this solution may be about 5.4. The pH of the solution may be adjusted with an acid or a base, or a pH adjuster. The pH adjuster may be sodium bicarbonate. The solution may be referred to as Ringer's solution.

In embodiments, the solution comprising the self-assembling peptide may comprise or may have added to it a contrast agent. The contrast agent may be utilized for visualization of the solution comprising the self-assembling peptide or the hydrogel or hydrogel barrier. The contrast agent may provide for or assure a practitioner the location of the solution comprising the self-assembling peptide or the hydrogel or hydrogel barrier. The contrast agent may comprise at least one of sulfate ions and sodium ions.

In embodiments, the properties of various self-assembling peptides, including but not limited to (RADA)₄ (SEQ ID NO: 1), (IEIK)₃I (SEQ ID NO: 2), (KLDL)₃ (SEQ ID NO: 3) may be enhanced by maintaining their salt concentration at less than their critical ionic strength level before they begin to precipitate. The critical ionic strength level of salts varies depending on the intrinsic amino acid characteristics and composition in each peptide. The peptides may be dissolved in water with various salts instead of pure water to maintain their salt ionic strength at less than their critical ionic strength level before they begin to precipitate.

This may beneficially impart relatively stiffer properties or higher mechanical strength to the self-assembling peptide solution and hydrogels at various salt concentrations rendering them suitable for a broader range of applications in comparison to peptide hydrogels maintained at a zero salt concentration level. This may also beneficially impart fast gelation kinetics from peptide solution to peptide hydrogels upon environmental salt ionic strength change to over their critical ionic strength before precipitation such as physiological ionic strength, which may occur when the peptide solution is administered to physiological conditions, for example, a target area of the subject.

In accordance with one or more aspects, the properties of various peptide hydrogels, including but not limited to (RADA)₄ (SEQ ID NO: 1), (IEIK)₃I (SEQ ID NO: 2), (KLDL)₃ (SEQ ID NO: 3), may be enhanced by maintaining their pH level at an elevated value of about 3.5 or less and at the same time, their salt concentration at less than their critical ionic strength level before they precipitate.

In some embodiments, the solution comprising (RADA)₄ (SEQ ID NO: 1)has a pH of about 3.5. In some embodiments, the solution comprising (KLDL)₃ (SEQ ID NO: 3), has a pH of about 3.5. In some embodiments, the solution comprising (IEIK)₃I (SEQ ID NO: 2), has a pH of about 3.7.

In some embodiments, a buffer, such as a buffer solution may be added to the self-assembling peptide solution or the self-assembling peptide.

A buffer may be an aqueous solution consisting of a mixture of a weak acid and its conjugate base, or vice versa. The pH of the buffer changes very little when a small or moderate amount of strong acid or base is added to it and thus it is used to prevent changes in the pH of a solution. Buffer solutions are used as a means of keeping pH at a nearly constant value in a wide variety of chemical applications, and is applicable to the self-assembling peptides and self-assembling peptide solutions and compositions disclosed herein.

A buffer may comprise at least two salts. A buffer may have a pH of about 7.4, such as PBS buffer (phosphate buffered saline). A buffer may have a pH of about 7.2, such as DMEM buffer. In some embodiments, the buffer may be an alkali buffer.

In some embodiments, a solution or composition of the self-assembling peptide may be buffered with about 0.15 M of at least one of sodium chloride, potassium chloride, and calcium chloride. When the self-assembling peptide is (RADA)₄ (SEQ ID NO: 1), the buffer may comprise between about 0.6 and about 1.2 M of a salt. When the self-assembling peptide is (IEIK)₃I (SEQ ID NO: 2), the buffer may comprise between about 0.6 and about 1.2 M of a salt. When the self-assembling peptide is (RADA)₄ (SEQ ID NO: 1), the buffer may comprise between about 0.02 and about 0.04 M of a salt. When the self-assembling peptide is (KLDL)₃ (SEQ ID NO: 3), the buffer may comprise between about 0.1 and about 0.4 M of a salt.

In certain embodiments, methods of treatment such as methods of providing a bronchial obstruction are provided that further comprise selecting a salt to provide a predetermined mechanical strength to the solution. The method may further comprise selecting the concentration of the salt to provide the predetermined mechanical strength to the solution. The method may comprise selecting a salt to provide a predetermined ionic strength to the solution. The method may further comprise selecting the concentration of the salt to provide the predetermined ionic strength to the solution. The method may comprise selecting a salt to provide a predetermined pH to the solution. The method may further comprise selecting the concentration of the salt to provide the predetermined pH to the solution.

Additional peptides comprising one or more biologically or physiologically active amino acid sequences or motifs may be included as one of the components along with the self-assembling peptide. Other components may include biologically active compounds such as a drug or other treatment that may provide some benefit to the subject. For example, a cancer treating drug or anticancer drug may be administered with the self-assembling peptide, or may be administered separately.

The peptide, peptide solution, or hydrogel may comprise small molecular drugs to treat the subject or to prevent hemolysis, inflammation, and infection. The small molecular drugs may be selected from the group consisting of glucose, saccharose, purified saccharose, lactose, maltose, trehalose, dextran, iodine, lysozyme chloride, dimethylisoprpylazulene, tretinoin tocoferil, povidone iodine, alprostadil alfadex, anise alcohol, isoamyl salicylate, α,α-dimethylphenylethyl alcohol, bacdanol, helional, sulfazin silver, bucladesine sodium, alprostadil alfadex, gentamycin sulfate, tetracycline hydrochloride, sodium fusidate, mupirocin calcium hydrate and isoamyl benzoate. Other small molecular drugs may be contemplated. Protein-based drugs may be included as a component to be administered, and may include erythropoietin, tissue type plasminogen activator, synthetic hemoglobin and insulin.

A component may be included to protect the self-assembling peptide, the solution comprising the self-assembling peptide, or the composition against rapid or immediate formation into a hydrogel. This may include an encapsulated delivery system that may degrade over time to allow a controlled time release of the peptide solution into the target area to form the hydrogel over a desired, predetermined period of time. Biodegradable, biocompatible polymers may be used, such as ethylene vinyl acetate, polyanhydrides, polyglycolic acid, collagen, polyorthoesters, and polylactic acid.

Any of the components described herein may be included in the self-assembling peptide, the solution comprising the self-assembling peptide, the composition, or kit or may be administered separate from the self-assembling peptide, the solution comprising the self-assembling peptide, the composition, or the kit. Additionally, any of the methods and methods of facilitating provided herein may be performed by one or more parties.

A peptide, peptide solution, composition or hydrogel of the disclosure may be provided in a kit for forming a bronchial obstruction, for example, a lung volume reduction. The kit may be for providing a bronchial obstruction in a subject. Instructions for administering a solution of self-assembling peptide to a target area of a subject may also be provided in the kit. The self-assembling peptide may comprise between about 7 amino acids and about 32 amino acids in an effective amount to form a hydrogel barrier to provide a bronchial obstruction. In some embodiments, the self-assembling peptide may comprise, consist of, or consist essentially between about 12 and about 16 amino acids. The self-assembling peptide may comprise, consist essentially of, or consist of (RADA)₄ (SEQ ID NO: 1), (IEIK)₃I (SEQ ID NO: 2), (KLDL)₃ (SEQ ID NO: 3). The concentrations of the self-assembling peptide in solution may be any of the concentrations disclosed herein.

The instructions for administering the solution may comprise methods for administering the peptide, peptide solution, or hydrogel provided herein, for example, by a route of administration described herein, at a dose, volume or concentration, or administration schedule. The peptide may be amphiphilic and at least a portion of the peptide may alternate between a hydrophobic amino acid and a hydrophilic amino acid.

The kit may provide the self-assembling peptide as one of a solution comprising a self-assembling peptide and a powder to be prepared as a solution comprising a self-assembling peptide. Instructions for preparing a solution comprising a self-assembling peptide having an effective concentration to form a hydrogel barrier under physiological conditions to form a bronchial obstruction may also be provided.

The kit may also comprise informational material. The informational material may be descriptive, instructional, marketing or other material that relates to the methods described herein. In one embodiment, the informational material may include information about production of the peptide, peptide solution, or hydrogel disclosed herein, physical properties of the peptide, composition, peptide solution or hydrogel, concentration, volume, size, dimensions, date of expiration, and batch or production site.

The kit may also optionally include a device or materials to allow for administration of the peptide or peptide solution to the desired area. For example, a syringe, pipette, catheter, or other needle-based device may be included in the kit. Additionally, or alternatively, the kit may include a guidewire, endoscope, or other accompanying equipment to provide selective administration of the peptide solution to the target area. In some embodiments, an endotracheal tube combined with a catheter to prevent backflow during injection may be provided.

The kit may comprise in addition to or in the alternative, other components or ingredients, such as components that may aid in positioning of the peptide solution, hydrogel or scaffold. Instructions may be provided in the kit to combine a sufficient quantity or volume of the peptide solution with a sucrose solution, that may or may not be provided with the kit. Instructions may be provided for diluting the peptide solution to administer an effective concentration of the solution to the target area. The instruction may describe diluting the peptide solution with a diluent or solvent. The diluent or solvent may be water. Instructions may further be provided for determining at least one of the effective concentration of the solution and the effective amount of the solution to the target area. This may be based on various parameters discussed herein, and may include the dimensions of the target area where a bronchial obstruction is desired.

Other components or ingredients may be included in the kit, in the same or different compositions or containers than the peptide, peptide solutions, or hydrogel. The one or more components that may include components that may provide for enhanced effectiveness of the self-assembling peptide or may provide another action, treatment, therapy, or otherwise interact with one or more components of the subject. For example, additional peptides comprising one or more biologically or physiologically active sequences or motifs may be included as one of the components along with the self-assembling peptide. Other components may include biologically active compounds such as a drug or other treatment that may provide some benefit to the subject. For example, a cancer treating drug or anticancer drug may be administered with the self-assembling peptide, or may be administered separately. The peptide, peptide solution, or hydrogel may comprise small molecular drugs to treat the subject or to prevent hemolysis, inflammation, and infection, as disclosed herein. A sugar solution such as a sucrose solution may be provided with the kit. The sucrose solution may be a 20% sucrose solution.

Other components which are disclosed herein throughout this disclosure may also be included in the kit. For example, the kit may further comprise salt solutions separate, or in combination with the self-assembling peptide. The kit may further comprise, for example, a sugar or sugar solution, for example, sucrose, that is provided separately from the self-assembling peptide or together with the self-assembling peptide. Instructions may be provided for combining a salt solution and one of the solution comprising the self-assembling peptide, or the peptide powder. The kit may further comprise an isotonic solution or contrast agent to be added to the self-assembling peptide solution or powder, or as part of the self-assembling peptide solution.

In some embodiments, a component of the kit is stored in a sealed vial, for example, with a rubber or silicone closure (for example, a polybutadiene or polyisoprene closure). In some embodiments, a component of the kit is stored under inert conditions (for example, under nitrogen or another inert gas such as argon). In some embodiments, a component of the kit is stored under anhydrous conditions (for example, with a desiccant). In some embodiments, a component of the kit is stored in a light blocking container such as an amber vial.

As part of the kit or separate from a kit, syringes or pipettes may be pre-filled with a peptide, peptide solution, or hydrogel as disclosed herein. Methods to instruct a user to supply a self-assembling peptide solution to a syringe or pipette, with or without the use of other devices, and administering it to the target area through the syringe or pipette, with or without the use of other devices, is provided. Other devices may include, for example, a catheter with or without a guidewire.

The self-assembling peptide of the kit may be any peptide provided in this disclosure for use as a bronchial obstruction agent, and any components described in this disclosure, for example, various salts, pH adjusters, buffers, alkali buffers may be provided in the kit, with the self-assembling peptide in the kit, or separately from the self-assembling peptide in the kit.

In embodiments, compositions comprising a self-assembling peptide comprising between about 7 amino acids and 32 amino acids in an effective amount and in an effective concentration is provided for use in forming a hydrogel barrier under physiological conditions to provide a bronchial obstruction. The self-assembling peptide of the composition may be selected from the group consisting of (RADA)₄ (SEQ ID NO: 1), (IEIK)₃I (SEQ ID NO: 2), and (KLDL)₃ (SEQ ID NO: 3). The concentration effective to allow formation of a bronchial obstruction comprises a self-assembling peptide concentration in a range of about 0.1 weight per volume (w/v) percent to about 3 w/v percent. The composition may be substantially free of cells. The composition may be substantially free of drugs. The composition may further comprise any one or more of the components disclosed herein. For example, the composition may comprise any one or more of the cations, anions, salts, buffers, contrast agents, isotonic solutions, pH adjusters, and sugars disclosed herein, and at the various concentration disclosed herein. The compositions may have properties such as mechanical strength, pH, gelation kinetics, and ionic strength as disclosed herein. The compositions may be used for providing a bronchial obstruction, such as but not limited to a lung volume reduction, and may be treatments for a subject, such as a mammal or human.

Method of facilitating formation of a bronchial obstruction in a subject may also be provided. The methods may comprise providing a solution comprising a self-assembling peptide comprising between about 7 amino acids to about 32 amino acids in an effective amount and in an effective concentration to form a hydrogel barrier under physiological conditions to allow formation of a bronchial obstruction; and providing instructions for administering the solution to a target area of the pulmonary system through introduction of the solution through a delivery device positioned in, for example, at least one lung lobe.

The methods may further comprise providing instructions to visualize a region comprising at least a portion of the bronchial system, such as a lobe of lung, as disclosed herein. Instructions may also be provided to visualize the region at which bronchial obstruction is desired, wherein the instructions comprise at least one of identifying the target area of the pulmonary system; introducing the delivery device; positioning an end of the delivery device in the target area; administering the solution; removing the delivery device from the target site; and monitoring the target site for obstruction after removing the delivery device. Instructions may be provided to visualize the region in a time period of about 1 minute to about 5 minutes subsequent the step of administering the solution. The method may further comprise providing instructions to prepare at least one of the effective amount and the effective concentration based in part on a dimension of the target area for bronchial obstruction, as discussed in the disclosure.

The self-assembling peptide is selected from the group consisting of (RADA)₄ (SEQ ID NO: 1), (IEIK)₃I (SEQ ID NO: 2), and (KLDL)₃ (SEQ ID NO: 3). The method may further comprise providing instructions to monitor the area surrounding the target area. The method may further comprise providing the solution and instructions for use before, during, or after a surgical procedure. The method comprising providing a solution comprising a self-assembling peptide may comprise providing instructions for preparing a peptide solution, as disclosed herein, having an effective concentration to form a hydrogel barrier under physiological conditions to allow for bronchial obstruction.

### EXAMPLES

### Example 1: Impact of pH level on rheological properties of peptide hydrogels

The effects of Dulbecco's modified Eagle's medium (DMEM) (pH 7.4) on the rheological properties of IEIK13, KLD12, and PuraMatrix® were evaluated on a rheometer (AR500, TA Instruments) with 40 mm plates. DMEM is generally a cell culture medium that contains 6.4 g/L of NaCl, 3.4 g/L NaHCO₃ (sodium bicarbonate), minor amounts of other salts, various amino acids, and 4.5 g/L of glucose. The pH level of DMEM is generally 7.2±0.2 and the osmolality is 335±30 mOsm/Kg H₂O; both measurements are close to human physiological fluids such as blood.

Peptide solutions (1%) were kept at 4 °C for at least 48 hours before testing. To perform the experiment, 1 mL of peptide solution was gently pipetted and placed on the plate of the rheometer. 2 mL of DMEM solution was gently added around the peptide solution. The peptide solution was treated with DMEM for two minutes, then the media was removed, and the plates were placed at a measuring geometry gap at around 450 µm. Measurements were performed at 37 °C after 2 min of relaxation time. Frequency tests were performed from 1 rad/s to 100 rad/s at 1 Pa of oscillation stress.

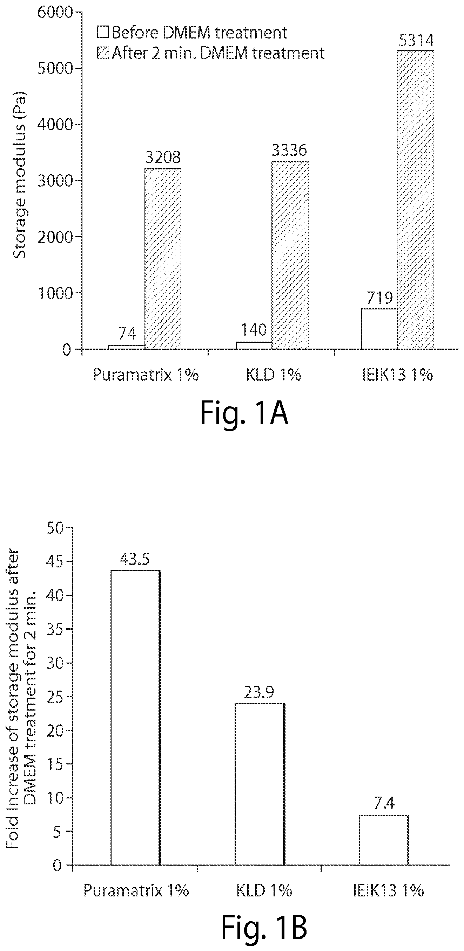

The rheological properties of the peptides (1%) were compared before and after DMEM treatment for 2 minutes, as shown in FIG. 1A. The fold increase of storage moduli after DMEM treatment for 2 minutes is shown in FIG. 1B. Each of the peptides showed large increases of storage moduli after DMEM treatment. The fold difference between storage moduli after DMEM treatment between PuraMatrix®, KLD12, and IEIK13 was relatively slight compared to that before DMEM treatment. Similarly, stiffer peptide solutions (i.e. IEIK13) showed lower-fold increase of storage modulus than weaker peptide solutions (i.e. PuraMatrix®) after DMEM treatment. This observation suggests that a critical intermolecular interaction arises after DMEM treatment, which determines the final stiffness after DMEM treatment.

### Example 2: Optimization of pH level of peptide solutions

To adjust the pH level of the peptide solutions by way of example, 0.1 N NaOH was added to 2 mL of 2.5% peptide solutions and their pH and appearance were measured. Results are shown in Table 1. Notably, a pH increase up to approximately 3.5 or less did not change the clear color of PuraMatrix®, IEIK13, and KLD12 solutions, while their apparent stiffness increased.

**Table 1 Appearance of peptide solutions at various pH levels**

| Peptides | 0.1 N NaOH added in 2.5% solution (µL/mL) | Conc. (%) | Peptide solution pH | Appearance |
|---|---|---|---|---|
| PuraMatrix® | 0 | 2.5 | 2.2 | Clear, thick gel |
| | 50 | 2.38 | 2.3 | Clear, thick gel |
| | 100 | 2.27 | 2.4 | Clear, thick gel |
| | 150 | 2.17 | 2.7 | Clear, thick, stiffer gel |
| | 200 | 2.08 | 2.9 | Clear, thick, stiffer gel |
| | 250 | 2.0 | 3.2 | Clear, thick, stiffer gel |
| | 275 | 1.96 | 3.4 | Clear, thick, stiffer gel |
| | 300 | 1.92 | 3.6 | Slightly cloudy, brittle gel |
| | 350 | 1.85 | 4.5 | Cloudy, phase-separated |
| | | | 7.0 | Cloudy, phase-separated |
| IEIK13 | 0 | 2.5 | 1.8 | Clear, thick gel |
| | 50 | 2.38 | 2.1 | Clear, thick gel |
| | 100 | 2.27 | 2.2 | Clear, thick gel |
| | 150 | 2.17 | 2.7 | Clear, thick gel, stiffer gel |
| | 200 | 2.08 | 3.0 | Clear, thick gel, stiffer gel |
| | 250 | 2.0 | 3.3 | Clear, thick gel, stiffer gel |
| | 275 | 1.96 | 3.7 | Clear, thick, stiffer gel |
| | 300 | 1.92 | 4.0 | Slightly cloudy, brittle gel |
| | 350 | 1.85 | 4.5 | Cloudy, brittle gel |
| | 400 | 1.79 | 5.4 | Cloudy, phase-separated |
| | | | 7.0 | Cloudy, phase-separated |
| KLD12 | 0 | 2.5 | 2.1 | Clear, thick gel |
| | 50 | 2.38 | 2.4 | Clear, thick gel |
| | 100 | 2.27 | 2.6 | Clear, thick gel |
| | 150 | 2.17 | 2.9 | Clear, thick and stiffer gel |
| | 200 | 2.08 | 3.3 | Clear, thick and stiffer gel |
| | 225 | 2.04 | 3.6 | Clear, thick and stiffer gel |
| | 250 | 2.0 | 4.0 | Slightly cloudy, brittle gel |
| | 300 | 1.92 | 4.7 | Cloudy, brittle gel |
| | 350 | 1.85 | 5.2 | Cloudy, phase-separated |
| | | | 7.0 | Cloudy, phase-separated |

### Example 3: Rheological properties of pH adjusted peptide solutions

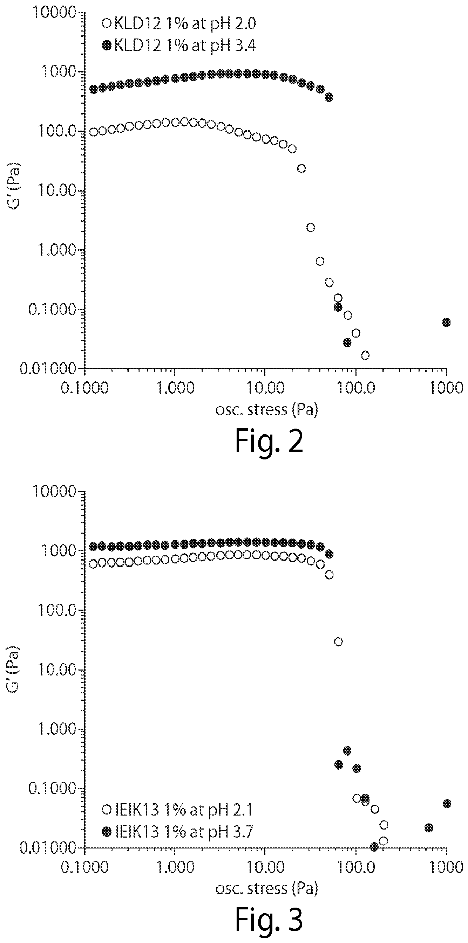

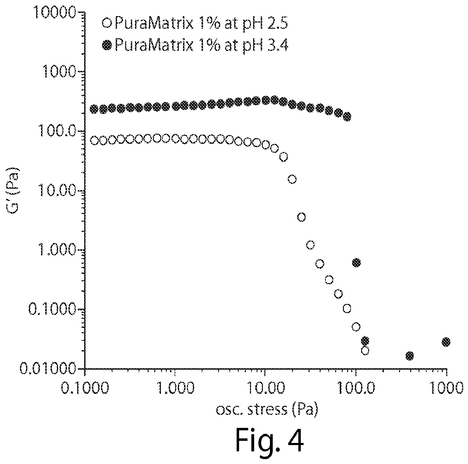

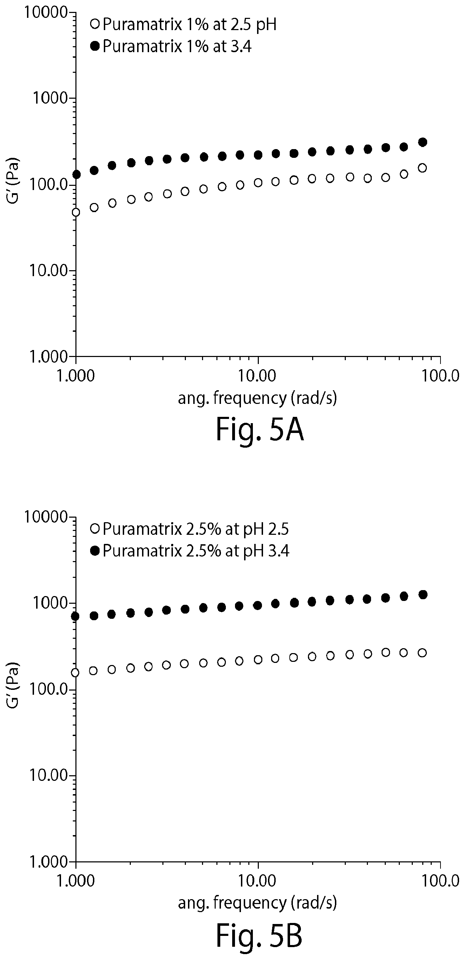

Based on the visual observation of the effect of pH level on the properties of the peptide solutions, the effect on the rheological properties of the peptide solutions after adjusting their pH level to 3.4 (PuraMatrix® and KLD12) or 3.7 (IEIK13) was evaluated. If the pH levels of peptide solutions are higher than 3.5 (PuraMatrix® and KLD12) or 3.7 (IEIK13), the peptide solution began phase separation becoming cloudy. The rheological properties of PuraMatrix®, KLD12 and IEIK13 solutions were higher at pH 3.4. The results are shown in FIG. 2 for KLD12 1%, FIG. 3 for IEIK13 1%, and FIGS. 4-5 for PuraMatrix® 1% and 2.5%, respectively. Stress sweep tests were performed at 10 rad/s. Frequency sweep tests were performed at 1 Pa.

### Example 4: Further rheological properties of pH adjusted peptide solutions

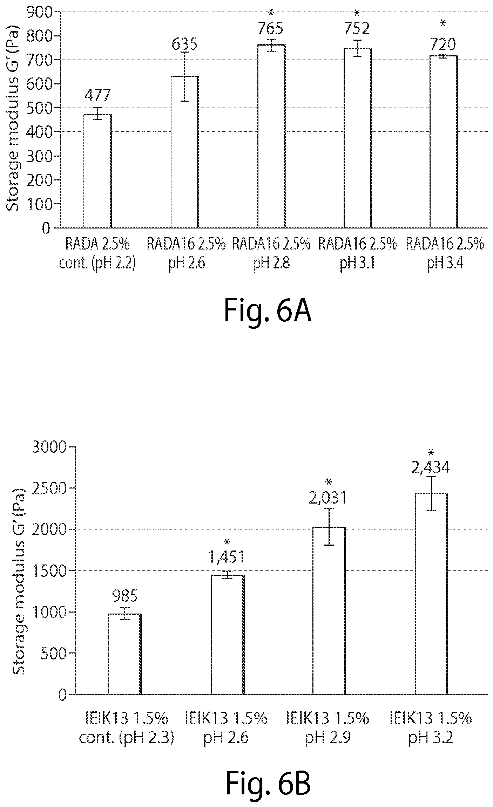

Based on the results of the effect on the rheological properties of the peptide solutions after adjusting their pH level to 3.4 (PuraMatrix® and KLD12) or 3.7 (IEIK13), the effect on the rheological properties of the peptide solutions at various pH levels was evaluated. The rheological property of PuraMatrix® and IEIK13 solutions increases with pH adjustment up to 3.4. The rheological properties of peptides were evaluated at various concentrations using a rheometer (DHR-1, TA Instruments) with 20 mm plates. The results are shown in FIG. 6A for PuraMatrix® 2.5% solution and FIG. 6B for IEIK13 1.5% solution, respectively. Frequency sweep tests were performed from 1 rad/sec to 10 rad/sec at 1 Pa and the storage modulus at 1 rad/sec was selected for data.

### Example 5: Effect of pH level on rheological properties of peptide hydrogels at various concentrations before/after DMEM treatment

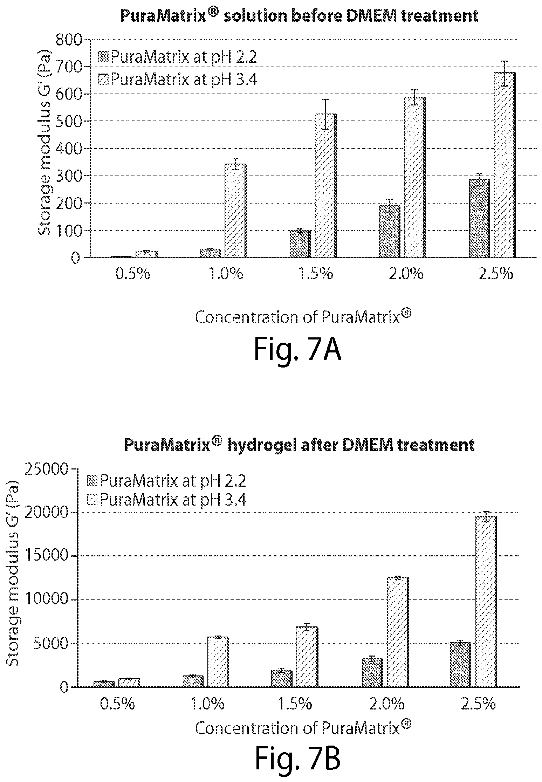

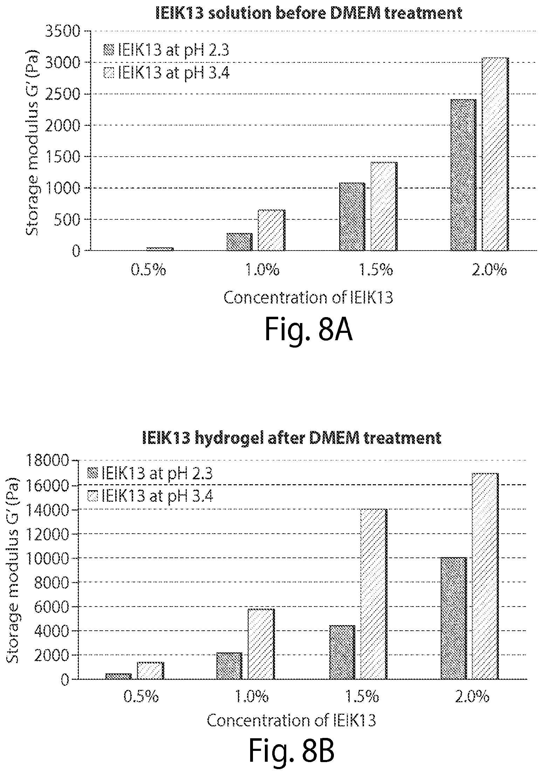

Based on the results of the effect on the rheological properties of the peptide solutions after adjusting their pH level, the effect on the rheological properties of the peptide hydrogels at various pH after DMEM treatment was evaluated and compared to the effect on the rheological properties of the peptide solutions at various pH before DMEM treatment. The rheological property of PuraMatrix® and IEIK13 hydrogels after DMEM treatment increases with pH adjustment up to 3.4. The results are shown in FIGS. 7A-7B for PuraMatrix® and FIGS. 8A-8B for IEIK (SEQ ID NO: 5), respectively.. Frequency sweep tests were performed from 1 rad/sec to 10 rad/sec at 1 Pa and the storage modulus at 1 rad/sec was selected for data.

### Example 6: Effect on gelation kinetics of pH adjusted peptide hydrogels

The effect of pH level on the properties of gelation kinetics was evaluated to identify optimized pH levels for the peptides as described herein. Fast gelation kinetics of PuraMatrix® and other peptides within body fluid may generally improve its function and response time for various clinical applications. The pH level may impart response time to begin gelation when treated with simulated body fluid including but not limited to DMEM. PuraMatrix® without pH adjustment (pH 2.2) did not show a storage modulus increase for the initial 13 seconds, while PuraMatrix® with pH adjustment showed immediate storage modulus increase due to fast gelation. A fast response time of PuraMatrix® and other peptides within body fluid may generally improve its function and response time for various clinical applications.

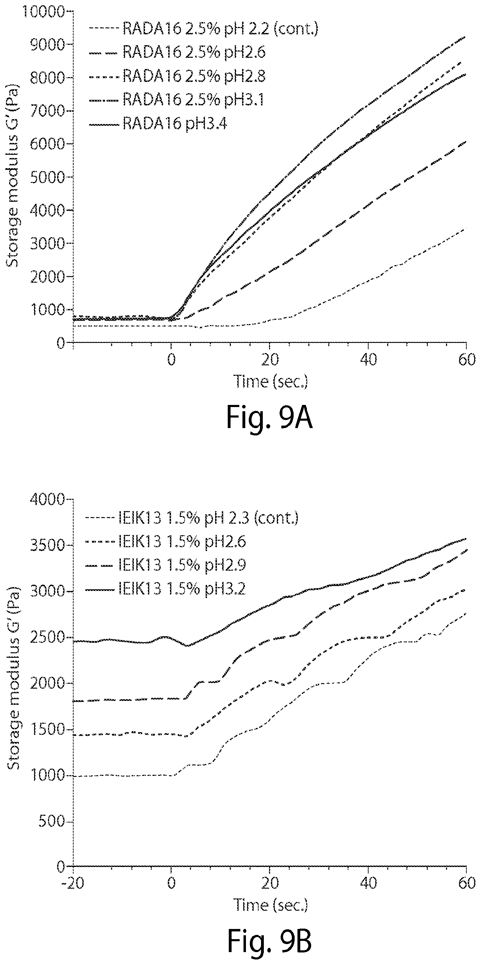

Time sweep tests were performed at 1 rad/sec and at 1 Pa with 20 mm plates and 500 µm gap distance. During time sweep test of PuraMatrix® 2.5% solution, DMEM was added into the chamber surrounding the measuring plates to soak PuraMatrix® solution at 0 time point. The results are shown in FIG. 9A for PuraMatrix® 2.5% solution.

IEIK (SEQ ID NO: 5) without pH adjustment showed an immediate storage modulus increase, while PuraMatrix® without pH adjustment (pH 2.2) did not show a storage modulus increase for the initial 13 seconds. IEIK13 with pH adjustment also showed an immediate storage modulus increase due to fast gelation. A fast response time of IEIK13 within body fluid may generally improve its function and response time for various clinical applications.

Time sweep tests were performed at 1 rad/sec and at 1 Pa with 20 mm plates and 500 µm gap distance. During time sweep test of IEIK13 1.5% solution, DMEM was added into the chamber surrounding the measuring plates to soak IEIK13 1.5% solution at 0 time point and continuously data was recorded. The results are shown in FIG. 9B for IEIK13 1.5% solution.

### Example 7: Effect of salt ionic strength level on peptide solutions and hydrogels

The effect of salt ionic strength level on the properties of peptide solutions was evaluated to identify optimized salt ionic strength levels for the peptides as described herein. Increasing the salt ionic strength level of PuraMatrix® and other peptides may generally improve its function and mechanical strength for various clinical applications. To adjust the salt ionic strength of the peptide solutions by way of example, various salt buffer solutions including NaCl, KCl, MgCl₂, CaCl₂ and DPBS (10X) were added to 2 mL of 1.5% peptide solutions.

Results are shown for PuraMatrix® in Table 2a. Notably, a salt ionic strength increase up to approximately 0.85 ∼1.15 M (depending on different salts) did not noticeably change the clear color of PuraMatrix® solutions, while their apparent stiffness increased. Results are shown for KLD12 in Table 2b. Notably, a salt ionic strength increase up to approximately 0.25∼0.35 M (depending on different salts) did not noticeably change the clear color of KLD12 solutions, while their apparent stiffness increased. Results are shown for IEIK13 in Table 2c. Notably, a salt ionic strength increase up to approximately 0.025∼0.035 M (depending on different salts) did not change the clear color of IEIK13 solutions, while their apparent stiffness increased.

**Table 2a Appearance of PuraMatrix® solution with various salts at room temperature**

| Salt solution | Volume of salt solution added in 1.5% PuraMatrix® solution (µL/mL) | Conc. of PuraMatrix® (%) | Conc. of salt (M) | Ionic Strength (M) | Appearance |
|---|---|---|---|---|---|
| NaCl (3 M-as a stock solution) | 0 | 1.5 | 0 | 0 | Clear, thick gel |
| | 52.6 | 1.43 | 0.15 | 0.15 | Clear, thick, stiffer gel |
| | 111.1 | 1.35 | 0.3 | 0.3 | Clear, thick, stiffer gel |
| | 176.5 | 1.27 | 0.45 | 0.45 | Clear, thick, stiffer gel |
| | 250 | 1.2 | 0.6 | 0.6 | Clear, thick, stiffer gel |
| | 333.3 | 1.13 | 0.75 | 0.75 | Clear, thick, stiffer gel |
| | 363.6 | 1.10 | 0.8 | 0.8 | Clear, thick, stiffer gel |
| | 395.3 | 1.08 | 0.85 | 0.85 | Clear, thick, stiffer gel |
| | 428.6 | 1.05 | 0.9 | 0.9 | Slightly cloudy, brittle gel |
| | 463.4 | 1.03 | 0.95 | 0.95 | Cloudy, phase-separated |
| | 500 | 1.0 | 1.0 | 1.0 | Cloudy, phase-separated |
| KCl (3 M-as a stock solution) | 0 | 1.5 | 0 | 0 | Clear, thick gel |
| | 52.6 | 1.43 | 0.15 | 0.15 | Clear, thick, stiffer gel |
| | 111.1 | 1.35 | 0.3 | 0.3 | Clear, thick, stiffer gel |
| | 176.5 | 1.27 | 0.45 | 0.45 | Clear, thick, stiffer gel |
| | 250 | 1.2 | 0.6 | 0.6 | Clear, thick, stiffer gel |
| | 333.3 | 1.13 | 0.75 | 0.75 | Clear, thick, stiffer gel |
| | 428.6 | 1.05 | 0.9 | 0.9 | Clear, thick, stiffer gel |
| | 463.4 | 1.03 | 0.95 | 0.95 | Clear, thick, stiffer gel |
| | 500 | 1.0 | 1.0 | 1.0 | Clear, thick, stiffer gel |
| | 538.5 | 0.98 | 1.05 | 1.05 | Slightly cloudy, thick, stiffer gel |
| | 578.9 | 0.95 | 1.1 | 1.1 | Slightly cloudy, brittle gel |
| | 621.6 | 0.93 | 1.15 | 1.15 | Cloudy, phase-separated |
| MgCl₂ (3 M-as a stock solution) | 0 | 1.5 | 0 | 0 | Clear, thick gel |
| | 16.9 | 1.48 | 0.05 | 0.15 | Clear, thick, stiffer gel |
| | 34.5 | 1.45 | 0.1 | 0.3 | Clear, thick, stiffer gel |
| | 52.6 | 1.43 | 0.15 | 0.45 | Clear, thick, stiffer gel |
| | 71.4 | 1.4 | 0.2 | 0.6 | Clear, thick, stiffer gel |
| | 90.9 | 1.38 | 0.25 | 0.75 | Clear, thick, stiffer gel |
| | 111.1 | 1.35 | 0.3 | 0.9 | Clear, thick, stiffer gel |
| | 132.1 | 1.32 | 0.35 | 1.05 | Clear, thick, stiffer gel |
| | 146.5 | 1.31 | 0.383 | 1.15 | Clear, thick, stiffer gel |
| | 153.8 | 1.3 | 0.4 | 1.2 | Slightly cloudy, thick, stiffer gel |
| | 161.3 | 1.29 | 0.417 | 1.25 | Slightly cloudy, brittle gel |
| | 168.8 | 1.28 | 0.433 | 1.3 | Cloudy, phase-separated |
| CaCl₂ (3 M-as a stock solution) | 0 | 1.5 | 0 | 0 | Clear, thick gel |
| | 16.9 | 1.48 | 0.05 | 0.15 | Clear, thick, stiffer gel |
| | 34.5 | 1.45 | 0.1 | 0.3 | Clear, thick, stiffer gel |
| | 52.6 | 1.43 | 0.15 | 0.45 | Clear, thick, stiffer gel |
| | 71.4 | 1.4 | 0.2 | 0.6 | Clear, thick, stiffer gel |
| | 90.9 | 1.38 | 0.25 | 0.75 | Clear, thick, stiffer gel |
| | 111.1 | 1.35 | 0.3 | 0.9 | Clear, thick, stiffer gel |
| | 132.1 | 1.32 | 0.35 | 1.05 | Clear, thick, stiffer gel |
| | 146.5 | 1.31 | 0.383 | 1.15 | Clear, thick, stiffer gel |
| | 153.8 | 1.3 | 0.4 | 1.2 | Slightly cloudy, thick, stiffer gel |
| | 161.3 | 1.29 | 0.417 | 1.25 | Slightly cloudy, brittle gel |
| | 168.8 | 1.28 | 0.433 | 1.3 | Cloudy, phase-separated |
| DPBS (pH 3.2) (10X-1.5 M -as a stock solution) | 0 | 1.5 | 0 | 0 | Clear, thick gel |
| | 111.1 | 1.35 | 0.15 | 0.15 | Clear, thick, stiffer gel |
| | 250 | 1.2 | 0.3 | 0.3 | Clear, thick, stiffer gel |
| | 428.6 | 1.05 | 0.45 | 0.45 | Clear, thick, stiffer gel |
| | 666.7 | 0.9 | 0.6 | 0.6 | Clear, thick, stiffer gel |
| | 1000 | 0.75 | 0.75 | 0.75 | Clear, thick, stiffer gel |
| | 1500 | 0.6 | 0.9 | 0.9 | Clear, thick, stiffer gel |
| | 1725 | 0.55 | 0.95 | 0.95 | Slightly cloudy, brittle gel |
| | 2000 | 0.5 | 1.0 | 1.0 | Cloudy, phase-separated |

**Table 2b Appearance of KLD12 solution with various salts at room temperature**

| Salt solution | Volume of salt solution added in 1.5% KLD12 solution (µL/mL) | Conc. of PuraMatrix® (%) | Conc. of salt (M) | Ionic Strength (M) | Appearance |
|---|---|---|---|---|---|
| NaCl (3M-as a stock solution) | 0 | 1.5 | 0 | 0 | Clear, thick gel |
| | 16.9 | 1.48 | 0.05 | 0.5 | Clear, thick, stiffer gel |
| | 34.5 | 1.45 | 0.1 | 0.1 | Clear, thick, stiffer gel |
| | 52.6 | 1.43 | 0.15 | 0.15 | Clear, thick, stiffer gel |
| | 71.4 | 1.4 | 0.2 | 0.2 | Clear, thick, stiffer gel |
| | 90.9 | 1.38 | 0.25 | 0.25 | Clear, thick, stiffer gel |
| | 111.1 | 1.35 | 0.3 | 0.3 | Slightly cloudy, thick, stiffer gel |
| | 132.1 | 1.32 | 0.35 | 0.35 | Slightly cloudy, brittle gel |
| | 153.8 | 1.3 | 0.4 | 0.4 | Cloudy, phase-separated |
| KCl (3M-as a stock solution) | 0 | 1.5 | 0 | 0 | Clear, thick gel |
| | 16.9 | 1.48 | 0.05 | 0.5 | Clear, thick, stiffer gel |
| | 34.5 | 1.45 | 0.1 | 0.1 | Clear, thick, stiffer gel |
| | 52.6 | 1.43 | 0.15 | 0.15 | Clear, thick, stiffer gel |
| | 71.4 | 1.4 | 0.2 | 0.2 | Clear, thick, stiffer gel |
| | 90.9 | 1.38 | 0.25 | 0.25 | Clear, thick, stiffer gel |
| | 111.1 | 1.35 | 0.3 | 0.3 | Slightly cloudy, thick, stiffer gel |
| | 132.1 | 1.32 | 0.35 | 0.35 | Slightly cloudy, brittle gel |
| | 153.8 | 1.3 | 0.4 | 0.4 | Cloudy, phase-separated |
| MgCl₂ (3M-as a stock solution) | 0 | 1.5 | 0 | 0 | Clear, thick gel |
| | 16.9 | 1.48 | 0.05 | 0.15 | Clear, thick, stiffer gel |
| | 22.7 | 1.47 | 0.067 | 0.2 | Clear, thick, stiffer gel |
| | 28.6 | 1.46 | 0.083 | 0.25 | Clear, thick, stiffer gel |
| | 34.5 | 1.45 | 0.1 | 0.3 | Clear, thick, stiffer gel |
| | 40.2 | 1.44 | 0.117 | 0.35 | Clear, thick, stiffer gel |
| | 46.5 | 1.43 | 0.133 | 0.4 | Slightly cloudy, thick, stiffer gel |
| | 52.6 | 1.43 | 0.15 | 0.45 | Slightly cloudy, brittle gel |
| | 58.8 | 1.42 | 0.167 | 0.5 | Cloudy, phase-separated |
| CaCl₂ (3M-as a stock solution) | 0 | 1.5 | 0 | 0 | Clear, thick gel |
| | 16.9 | 1.48 | 0.05 | 0.15 | Clear, thick, stiffer gel |
| | 22.7 | 1.47 | 0.067 | 0.2 | Clear, thick, stiffer gel |
| | 28.6 | 1.46 | 0.083 | 0.25 | Clear, thick, stiffer gel |
| | 34.5 | 1.45 | 0.1 | 0.3 | Clear, thick, stiffer gel |
| | 40.2 | 1.44 | 0.117 | 0.35 | Clear, thick, stiffer gel |
| | 46.5 | 1.43 | 0.133 | 0.4 | Slightly cloudy, thick, stiffer gel |
| | 52.6 | 1.43 | 0.15 | 0.45 | Slightly cloudy, brittle gel |
| | 58.8 | 1.42 | 0.167 | 0.5 | Cloudy, phase-separated |

**Table 2c Appearance of IEIK13 solution with various salts at room temperature**

| Salt solution | Volume of salt solution added in 1.5% IEIK13 solution (µL/mL) | Conc. of PuraMatrix® (%) | Conc. of salt (M) | Ionic Strength (M) | Appearance |
|---|---|---|---|---|---|
| NaCl (0.2 M-as a stock solution) | 0 | 1.5 | 0 | 0 | Clear, thick gel |
| | 25.6 | 1.46 | 0.005 | 0.005 | Clear, thick, stiffer gel |
| | 52.6 | 1.43 | 0.01 | 0.01 | Clear, thick, stiffer gel |
| | 81.1 | 1.39 | 0.015 | 0.015 | Clear, thick, stiffer gel |
| | 111.1 | 1.35 | 0.02 | 0.02 | Clear, thick, stiffer gel |
| | 142.9 | 1.31 | 0.025 | 0.025 | Clear, thick, stiffer gel |
| | 176.5 | 1.27 | 0.03 | 0.03 | Slightly cloudy, thick, stiffer gel |
| | 212.1 | 1.24 | 0.035 | 0.035 | Slightly cloudy, brittle gel |
| | 250 | 1.2 | 0.04 | 0.04 | Cloudy, phase-separated |
| KCl (0.2 M-as a stock solution) | 0 | 1.5 | 0 | 0 | Clear, thick gel |
| | 25.6 | 1.46 | 0.005 | 0.005 | Clear, thick, stiffer gel |
| | 52.6 | 1.43 | 0.01 | 0.01 | Clear, thick, stiffer gel |
| | 81.1 | 1.39 | 0.015 | 0.015 | Clear, thick, stiffer gel |
| | 111.1 | 1.35 | 0.02 | 0.02 | Clear, thick, stiffer gel |
| | 142.9 | 1.31 | 0.025 | 0.025 | Clear, thick, stiffer gel |
| | 176.5 | 1.27 | 0.03 | 0.03 | Clear, thick, stiffer gel |
| | 212.1 | 1.24 | 0.035 | 0.035 | Slightly cloudy, brittle gel |
| | 250 | 1.2 | 0.04 | 0.04 | Slightly cloudy, brittle |
| | 290.3 | 1.16 | 0.045 | 0.045 | Cloudy, phase-separated |
| MgCl₂ (0.2 M-as a stock solution) | 0 | 1.5 | 0 | 0 | Clear, thick gel |
| | 25.6 | 1.46 | 0.005 | 0.015 | Clear, thick, stiffer gel |
| | 34.5 | 1.45 | 0.0067 | 0.02 | Clear, thick, stiffer gel |
| | 43.5 | 1.44 | 0.0083 | 0.025 | Clear, thick, stiffer gel |
| | 52.6 | 1.43 | 0.01 | 0.03 | Clear, thick, stiffer gel |
| | 61.9 | 1.41 | 0.0117 | 0.035 | Clear, thick, stiffer gel |
| | 71.4 | 1.40 | 0.0133 | 0.04 | Slightly cloudy, thick, stiffer gel |
| | 81.1 | 1.39 | 0.015 | 0.045 | Slightly cloudy, stiffer gel |
| | 91.1 | 1.37 | 0.0167 | 0.05 | Slightly cloudy, brittle gel |
| | 100.9 | 1.36 | 0.0183 | 0.055 | Cloudy, phase-separated |
| CaCl₂ (0.2 M-as a stock solution) | 0 | 1.5 | 0 | 0 | Clear, thick gel |
| | 25.6 | 1.46 | 0.005 | 0.015 | Clear, thick, stiffer gel |
| | 34.5 | 1.45 | 0.0067 | 0.02 | Clear, thick, stiffer gel |
| | 43.5 | 1.44 | 0.0083 | 0.025 | Clear, thick, stiffer gel |
| | 52.6 | 1.43 | 0.01 | 0.03 | Clear, thick, stiffer gel |
| | 61.9 | 1.41 | 0.0117 | 0.035 | Clear, thick, stiffer gel |
| | 71.4 | 1.40 | 0.0133 | 0.04 | Slightly cloudy, thick, stiffer gel |
| | 81.1 | 1.39 | 0.015 | 0.045 | Slightly cloudy, thick, stiffer gel |
| | 91.1 | 1.37 | 0.0167 | 0.05 | Slightly cloudy, brittle gel |
| | 100.9 | 1.36 | 0.0183 | 0.055 | Cloudy, phase-separated |

The results from Tables 1a-1c show that the critical salt ionic strengths at which three peptides become cloudy is shown as follows: PuraMatrix® (0.9-1.2 M) > KLD13 (0.3-0.4 M) > IEIK13 (0.03∼0.04 M).

### Example 8: Effect of salt ionic strength level on rheological properties of peptide solutions

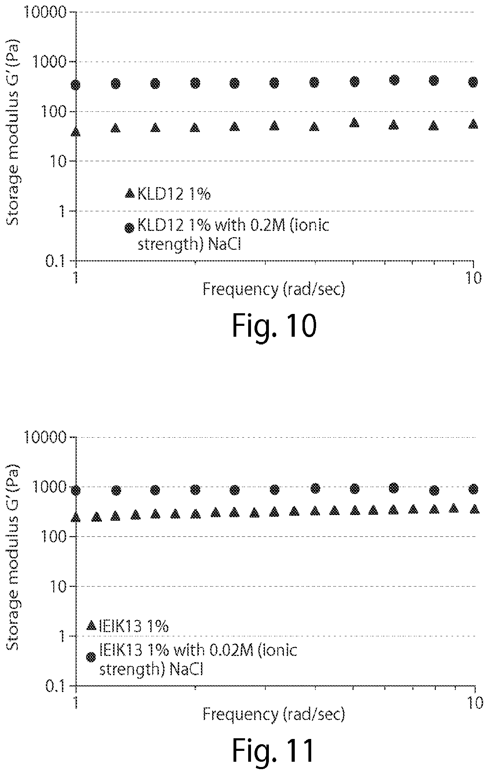

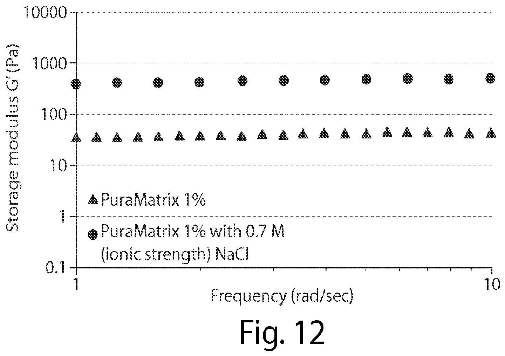

Based on the visual observation of the effect of salt ionic strength on the properties of the peptide solutions, the effect on the rheological properties of the peptide solutions after adjusting their ionic strength level with NaCl to 0.7 M (PuraMatrix®), 0.2 M (KLD12) or 0.02 M (IEIK13), which is closely below the critical ionic strength at which each peptide becomes cloudy, was evaluated. If the ionic strength levels with NaCl of peptide solutions are higher than 0.9 M (PuraMatrix®), 0.3 M (KLD12) or 0.03 M (IEIK13), the peptide solution begin phase separation becoming cloudy and weak. The rheological property of PuraMatrix®, KLD12 and IEIK13 solutions was higher after adjusting their ionic strength level with NaCl to 0.7 M (PuraMatrix®), 0.2 M (KLD12) or 0.02 M (IEIK13), The results are shown in FIG. 10 for KLD12 1%, FIG. 11 for IEIK13 1%, and FIG. 12 for PuraMatrix® 1%, respectively. Frequency sweep tests were performed from 1 rad/s to 10 rad/s at 1 Pa.

### Example 9: Further effect of salt ionic strength level on rheological properties of peptide solutions

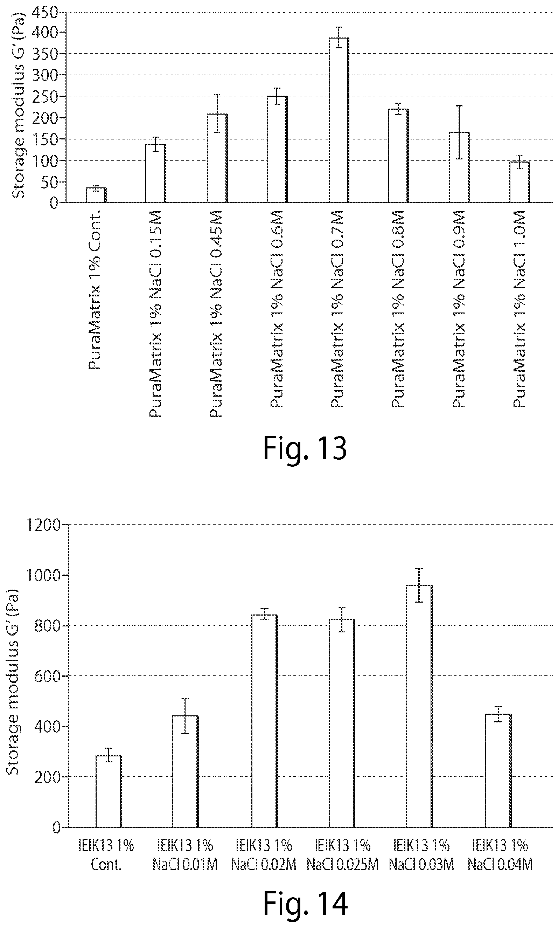

Based on the results of the effect on the rheological properties of the peptide solutions after adjusting their ionic strength level with NaCl to 0.7 M (PuraMatrix®), 0.2 M (KLD12) or 0.02 M (IEIK13), the effect on the rheological properties of the peptide solution at various salt ionic strengths was evaluated. The rheological property of PuraMatrix® 1% solutions increases with ionic strength adjustment up to 0.7 M, while decreases above 0.7 M. The rheological property of IEIK13 1% solutions increases with ionic strength adjustment up to 0.03 M, while decreases above 0.03 M. These results match well with visual inspection of the peptide solutions at various salt ionic strengths. The results are shown in FIG. 13 for PuraMatrix® 1% solution and in FIG. 14 for IEIK13 1% solution. Frequency sweep tests were performed from 1 rad/sec to 10 rad/sec at 1 Pa and the storage modulus at 1 rad/sec was selected for data.

### Example 10: Effect on rheological properties of peptide solutions after DMEM treatment

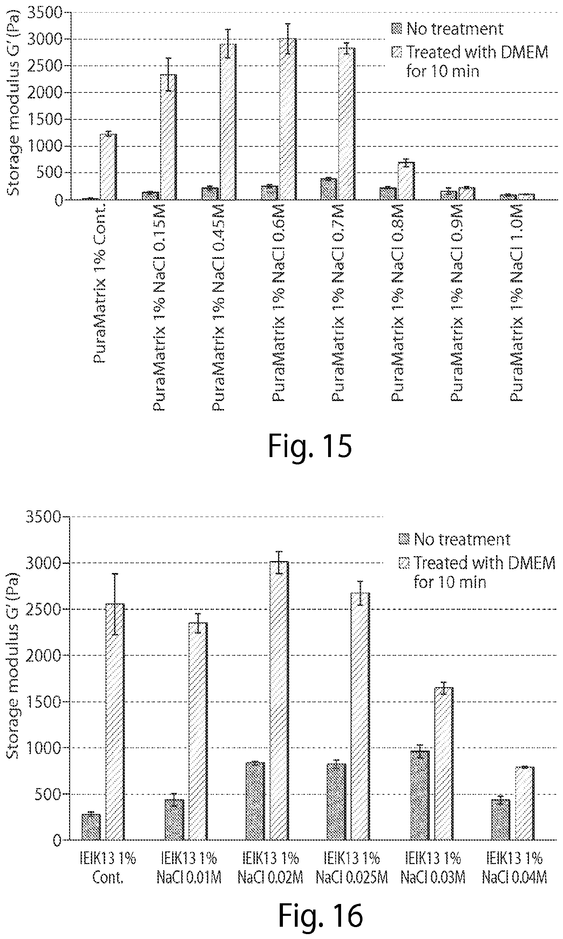

Based on the results of the effect on the rheological properties of the peptide solutions after adjusting their ionic strength levels, the effect on the rheological properties of the peptide hydrogels after DMEM treatment for 10 min was evaluated. The rheological property of PuraMatrix® hydrogels after DMEM treatment increased with ionic strength adjustment up to 0.7 M, while decreases above 0.7 M. The rheological property of IEIK13 hydrogels after DMEM treatment did not significantly change with ionic strength adjustment up to 0.025 M, while decreases above 0.03 M. Above 0.9 M of NaCl ionic strength at which PuraMatrix® solution becomes cloudy, the rheological properties of PuraMatrix® did not change with DMEM treatment, demonstrating there is no gelation. The results are shown in FIG. 15 for PuraMatrix® 1% hydrogels and in FIG. 16 for IEIK13 1% hydrogels, both after DMEM treatment for 10 min. Frequency sweep tests were performed from 1 rad/sec to 10 rad/sec at 1 Pa and the storage modulus at 1 rad/sec was selected for data.

### Example 11: Effect of various salts

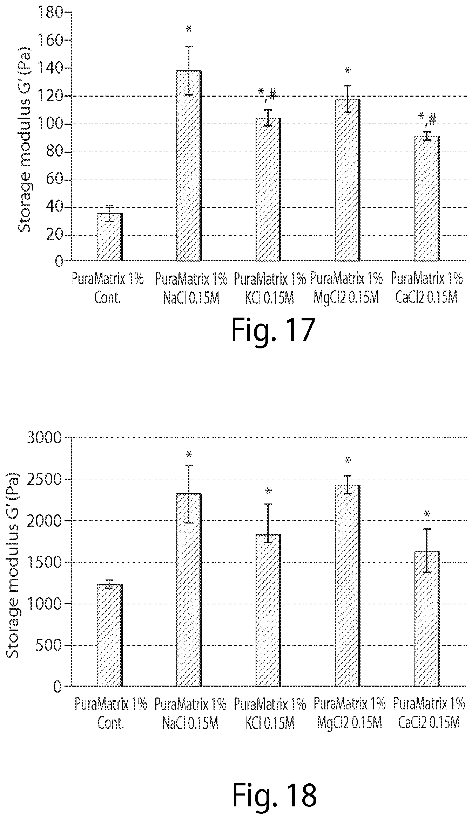

Based on the results of the effect on the rheological properties of the peptide solutions and hydrogels after adjusting their ionic strength levels with NaCl, the effect of various salts (KCl, MgCl₂, and CaCl₂) was also evaluated. The rheological property of PuraMatrix® solutions increases with ionic strength adjustment at 0.15 M of all the salts. Increases of the rheological property of PuraMatrix® solutions with various salts were not predominantly different. However, increases of the rheological property of PuraMatrix® solutions may vary depending on the salting out constant, K of each salt. Constant K is a constant in Cohen's equation: log S = B - KI, where S is solubility, B is idealized solubility, K is salting out constant, and I is ionic strength. With a higher value of constant K and ionic strength of salts, solubility of the peptide may decrease resulting in strong peptide self-assembly with increased hydrophobic effect and higher rheological properties of the peptide solution. The constant K of NaCl may be higher than the other salts. Thus, the rheological property of PuraMatrix® solutions with NaCl was slightly higher than those with KCl and CaCl₂. The rheological property of PuraMatrix® hydrogels after DMEM treatment for 10 min were also evaluated with ionic strength adjustment with various salts and the results were comparable to increases of the rheological property of PuraMatrix® solutions with various salts ((NaCl, KCL, MgCl₂, and CaCl₂) at 0.15 M ionic strength). The results are shown in FIG. 17 for PuraMatrix® 1% solution before DMEM treatment, and in FIG. 18 for PuraMatrix® 1% hydrogels after DMEM treatment for 10 min. Frequency sweep tests were performed from 1 rad/sec to 10 rad/sec at 1 Pa and the storage modulus at 1 rad/sec was selected for data. * denotes that data is significantly higher than PuraMatrix® control data (P < 0.05). # denotes that data is significantly lower than PuraMatrix® 1% NaCL 0.15M (ionic strength) data (P < 0.05).

### Example 12: Effect of various salts on gelation kinetics

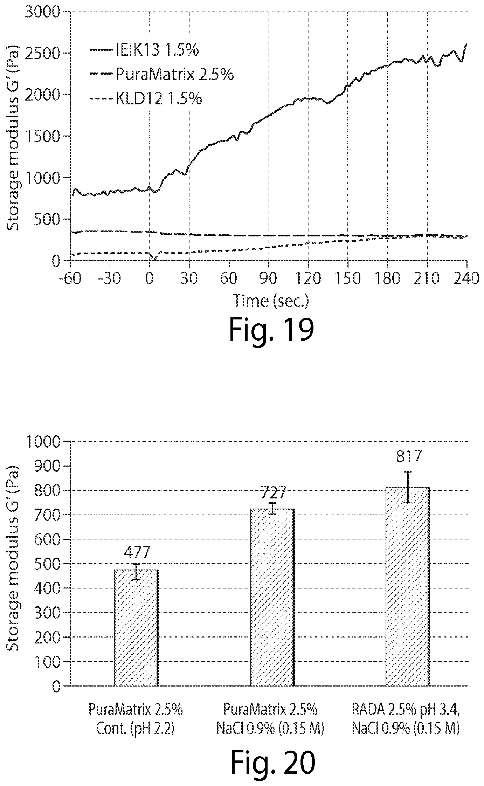

The effect of salt ionic strength level surrounding peptide solution on the properties of peptide solutions was evaluated to identify the possibility of peptide gelation when the peptide solution is placed into the environment where salt ionic strength level is high. For example, the hydrogels may be placed in the isotonic body fluid, which is comparable to saline buffer (0.15 M of NaCl). As demonstrated before, self-assembly peptides including but not limited to PuraMatrix®, KLD12 and IEIK13 form hydrogels when they are treated at neutral pH. Without pH effect, the effect of saline treatment on gelation of peptide solutions were evaluated. When peptide solutions were treated with saline buffer, their pH did not change. After saline buffer treatment, only IEIK13 showed fast gelation, while PuraMatrix® and KLD13 showed no or negligible gelation. This is because IEIK13 is much more sensitive to salt ionic strength levels. Fast gelation of IEIK13 at the salt ionic strength level similar to body fluid isotonic salt level may generally improve its function and gelation speed for various clinical applications. The results are shown in FIG. 19 for IEIK13, KLD12 and PuraMatrix® solutions. Time sweep tests were performed at 1 rad/sec and at 1 Pa with 20 mm plates and 500 µm gap distance. During time sweep test of IEIK13 1.5% , KLD12 1.5%, and PuraMatrix® 2.5% solution, DMEM was added into the chamber surrounding the measuring plates to soak PuraMatrix® solution at 0 time point.

### Example 13: Effect of salt ionic strength and pH adjustment on rheological properties

In accordance with one or more embodiments, IEIK13, KLD12, and PuraMatrix® may be dissolved both in salt buffer such as NaCl and at an elevated pH level adjusted with alkali salt buffer such as NaOH to keep their salt ionic strength under their critical salt points as well as their pH level to about 2.5∼4.0, so that they may have stiffer properties. With respect to PuraMatrix®, KLD13 and IEIK13, the peptide solutions are still clear with 0.9% NaCl (ionic strength: 0.15 M) at pH 3.4 adjusted with NaOH. The rheological property of PuraMatrix® with 0.9% NaCl (ionic strength: 0.15 M) at pH 3.4 was stiffer than those of PuraMatrix control and PuraMatrix with only NaCl 0.9%. The effect of salt ionic strength and pH adjustment on the rheological properties of PuraMatrix® 2.5 % solution are shown in FIG. 20. Frequency sweep tests were performed from 1 rad/sec to 10 rad/sec at 1 Pa and the storage modulus at 1 rad/sec was selected for data.

### Example 14: Influence of Cations

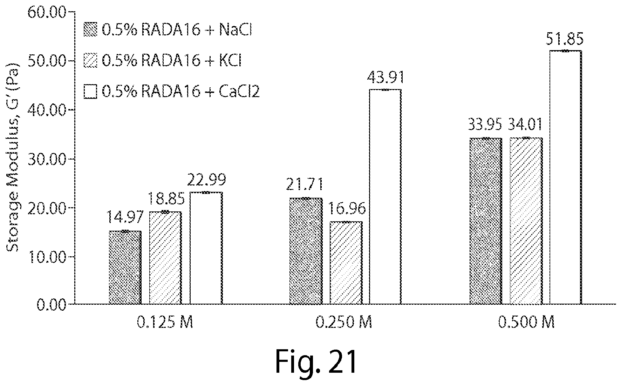

In a rheological comparison of 2.5% RADA16 and 2.5% RADA16 + NaCl, KCl, and CaCl2, solutions of 0.5% RADA16 mixed with 0.005, 0.05, 0.125, 0.25, 0.5, and 1 M NaCl, KCl, and CaCl2 were prepared. The anion, chloride (Cl-), was kept the same to observe the effect of the cations, sodium (Na+), potassium (K+), and calcium (Ca2+). FIG. 21 provides a basic understanding of how varying the cations of a salt solution effects the viscoelastic properties and the stiffness of self-assembling peptides. Ca provided the best enhancement of stiffness compared to either Na or K at the same molar concentrations. This should be because Ca has four times higher ionic strength than Na and K at the same molar concentrations. Therefore, influence of salts on the peptide solution is more related to their ionic strength rather than their molar concentration, as shown in FIGS. 17-18 and Table 2a-c. In some embodiments, there is a correlation between the properties of the peptide solutions with salts based on the concentration of the salt.

### Example 15: Mechanical strength

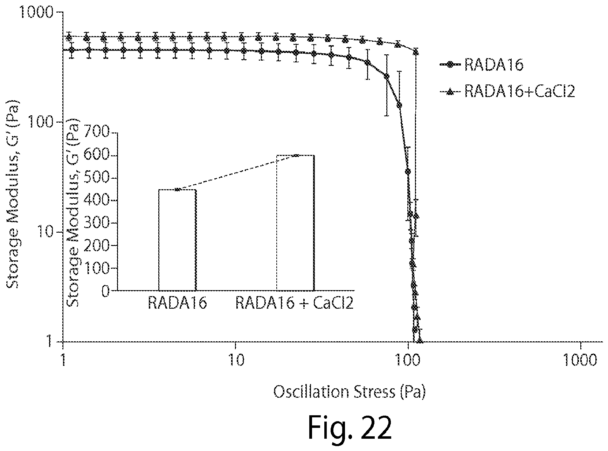

Rheological measurements of stiffness of 2.5% RADA16 and 2.5% RADA16 + 0.25 M CaCl2 was evaluated. FIG. 22 compares the stiffness of a high concentrated solution of RADA16 with another high concentrated solution of RADA16 with an addition of 0.125 M CaCl2 and provides a basic understanding of the viscoelastic properties of the peptide and peptide mixture. There was a noticeable increase in stiffness between the two solutions when a cation solution was added. Ca was shown to provide mechanical enhancement of RADA16 even at high concentrations using the optimal concentration range.

### Example 16: Reversibility

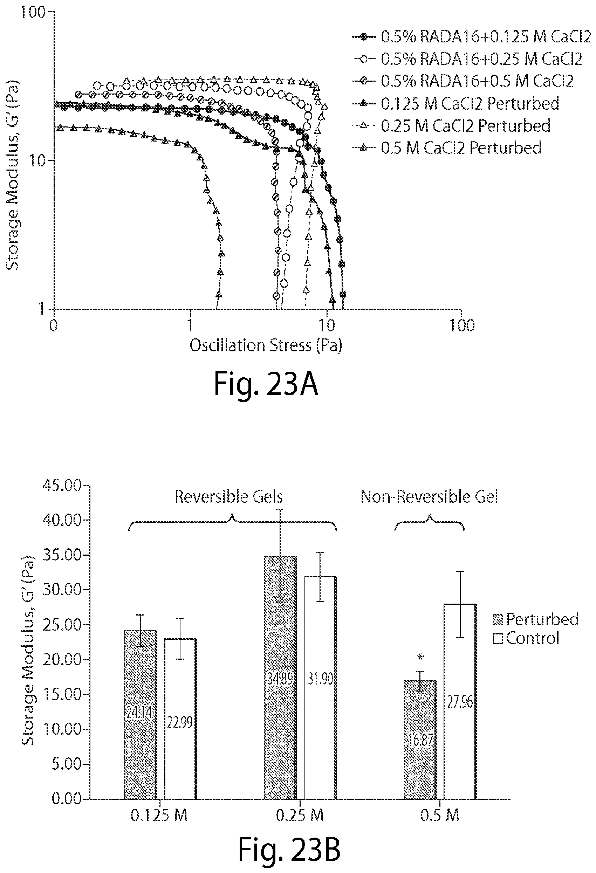

Rheological measurements of reversibility of 0.5% RADA16 solution with + 0.125, 0.25, and 0.5 M CaCl2 were evaluated. Solutions of 0.5% RADA16 mixed with 0.125, 0.25, and 0.5 M CaCl2 were prepared. The structure of the self-assembled peptide solutions were disrupted thoroughly by application of mechanical stress through vortexing and sonication. The mixtures were placed at room temperature for 48 hours to allow self-assembly to take place. FIGS. 23A-23B provide the basic viscoelastic properties of the peptide mixtures and show that reversibility of the peptide solution with salts can be controlled and maintained even after perturbation of the structure, specifically noted by the significant difference between the 2.5% RADA16 + 0.5 M CaCl2 control and perturbed samples. The mixtures within the optimal salt concentration range remained reversible. The * denotes the control sample and the perturbed sample, which follows, as being significantly different. FIG. 23a presents raw rheological data of the control peptide solution with salts and the perturbed peptide solution while FIG. 23b provides a comparison of stiffness of the control peptide solution and the perturbed peptide solution.

### Example 17: Gelation kinetics

Rheological measurements of gelation kinetics of 0.5% RADA16 + NaCl, KCl, and CaCl2 were evaluated. A solution of 0.5% RADA16 was prepared and gelation kinetics were observed by treatment with several cations (e.g. Na, Cl, K) and anions (e.g. Cl, CO3, PO4, SO4). It was determined how long it will take for the peptide mixtures to gel and how to control that gelation time by varying the cation/anion type and concentration. Chlorine showed the quickest gelation and sulfate showed the slowest gelation. *In vivo* and *in vitro* qualitative experiments and the resulting observations were supportive of these conclusions.

### Example 18: Varying cations

A peptide hydrogel mixed with a cation/anion solution which affected mechanical properties and another with a very low concentration of a contrast agent which did not affect the mechanical properties were both designed. The two gels were: (1) a combination of the self-assembling peptide with a well-known cation/anion solution, Ringer's Solution (pH 5.3), used in the medical field and (2) a combination of the self-assembling peptide with a well-known contrast agent, indigo carmine, which is a dye solution containing sulfate (anion) and sodium (cation) ions. Indigo carmine contains indigoindisulfonate sodium (C₁₆H₈N₂Na₂O₈S₂), water, and sodium citrate (C₆H₈O₇) for pH adjustment. Using indigo carmine powder, a 1% solution was prepared for use in experimentation. This corresponds to 10 mg/1 ml of water. The concentration of indigo carmine solution used in experimentation was 0.00585% in water.

Indigo carmine powder was used to prepare a 1% solution in deionized (DI) water. Using IEIK13 powder, a 2 percent solution was prepared using DI water. The amount of IEIK was weighed out and the appropriate amount of DI water was added gently down the side of the container. Mixing was accomplished by vortexing for about 30 seconds, and then sonicating for 30 seconds. The solution was then centrifuged for about 10 to about 15 minutes at 3000ppm. The solution may undergo further vortexing and centrifuging until the solution is clear and without bubbles.

To obtain a final concentration of 0.00585% indigo carmine, the necessary amount of 1% IC is added to the appropriate amount of DI water to dilute 2% IEIK to 1.5% IEIK. The solution is then vortexted for about 30 seconds and centrifuged for about 10 to about 15 minutes at 3000 rpm. The solution may undergo further vortexing and centrifuging until the solution is clear and without bubbles.

The solution was allowed to sit overnight at room temperature prior to use. The cap may be left off of the container during preparation to allow more efficient removal of bubbles.

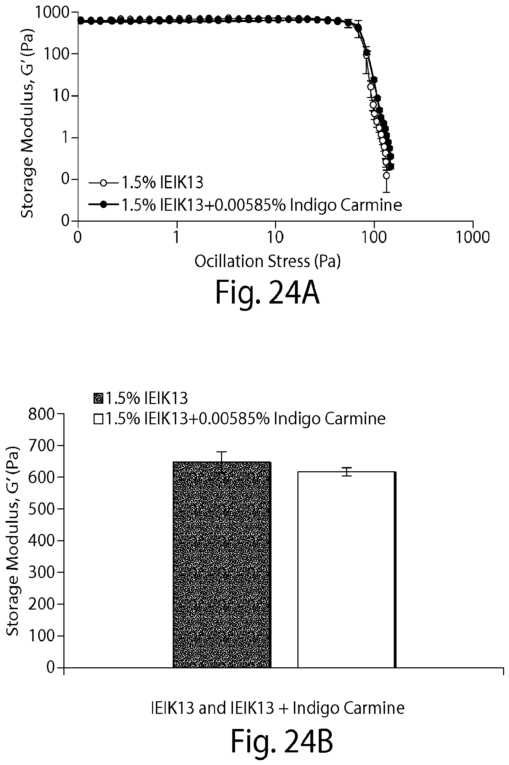

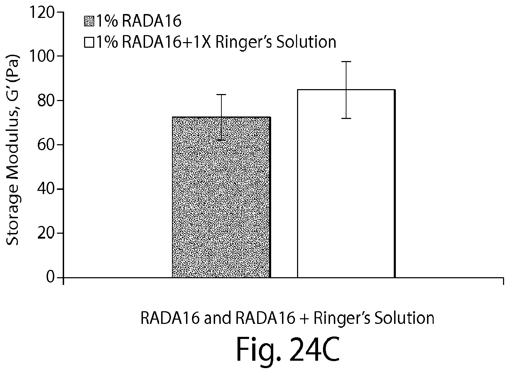

The rheological comparisons of these mixtures and the visualization of the gels can be observed in FIG. 24a-24c. A stiffer gel with faster gelation kinetics that maintains reversibility was obtained. Another that maintains stiffness, reversibility, and gelation kinetics, but allows for the dying of tissues for histology was also obtained. The concentration of the contrast agent or cation/anion mixture and peptide hydrogel that were mixed were based on an understanding of using cations and anions as described herein. The data relating to these tailored peptide hydrogels of RADA16 + Ringer's Solution and IEIK13 + Indigo Carmine show that these controlled self-assembling hydrogels can be tailored to fit the needs for an isotonic injectable gel and a non-mechanically enhanced gel for visualization. FIG. 24A presents raw rheological data of IEIK (SEQ ID NO: 5) and IEIK (SEQ ID NO: 5) mixed with Indigo Carmine. FIG. 24B shows a comparison of stiffness of IEIK (SEQ ID NO: 5) and IEIK (SEQ ID NO: 5) mixed with Indigo Carmine. FIG. 24C presents a comparison of stiffness of RADA16 and RADA16 mixed with Ringer's Solution.

### Example 19: Lung Volume Reduction

Injectable, self-assembling peptide hydrogel systems were applied to reduce the lung volume available for oxygen exchange in subjects. It was demonstrated that it is possible to fill at least a portion of a lung with peptide hydrogel to reduce lung volume.

### Materials and Methods

### Experimental Setup

Swine lungs were obtained from freshly euthanized pigs. An endotracheal tube, connected to a syringe filled with a peptide hydrogel, was inserted through the trachea and primary bronchus into the lobe of interest. The tube was led through the bronchi to the end of the appropriate lobe and the distance was noted.

### Preparation of Self-Assembling Peptide Hydrogels

The self-assembling peptide hydrogels were comprised of Ac-RADARADARADARADA-NH₂ (SEQ ID NO: 1) (i.e. RADA16) or Ac-IEIKIEIKIEIKI-NH₂ (SEQ ID NO: 2) (i.e. IEIK13). RADA16 or IEIK13 was mixed with 0.00585% Indigo Carmine to observe spreading of the hydrogels throughout the lobes. When mixed, the peptides were reconstituted with a salt solution. The peptides were reconstituted first in deionized water, and subsequently with 1% Indigo Carmine to provide a final concentration of 0.00585% of the contrast reagent.

### Application of Self-Assembling Peptide Hydrogels

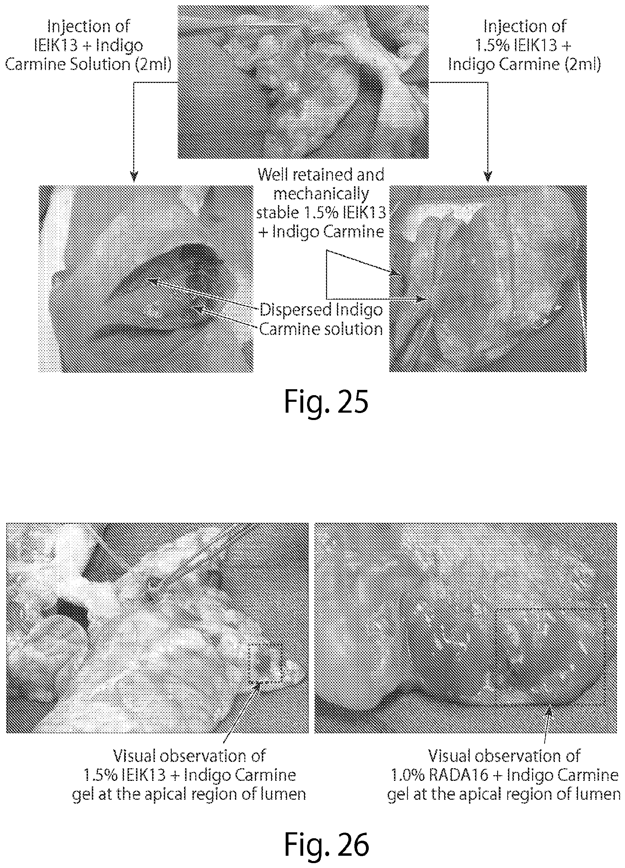

Once the lobe of interest was identified, the endotracheal tube was fed to the end where the hydrogels were injected. After each injection of 1 ml of hydrogel the tube was retracted 2 cm and the injection was repeated again. This procedure was repeated until a backflow of hydrogel was observed from the bronchi and the total volume of hydrogel injected was noted. The final injection ranged from 0.5 - 1.5 ml. After backflow of hydrogel was observed, the lobes were cut at 90 degrees to the midline for histological analysis. FIG. 25 illustrates the procedural flow of injecting peptide hydrogel into the bronchi of a single lobe of the lung and observation of penetration of the solution. Lumen penetration of 1% Indigo Carmine solution (left) was compared to 1.5% IEIK (SEQ ID NO: 5) + 0.00585% Indigo Carmine (right).

### Results

1.5% IEIK13 and 1.0% RADA16 with 0.00585% Indigo Carmine were used to identify the potential for lung volume reduction surgeries. 1% Indigo Carmine solution as well as an alternative product, Aeroseal, were used as comparisons to RADA16 and IEIK13. The hydrogels were applied to the lungs as noted above. 1.5% IEIK13 was applied several times, however, it was determined to be slightly too difficult to inject. 1.0% RADA16 was selected as a substitute to IEIK13 as its mechanical properties are much less which allows for greater spreading and penetration within the lobe. FIG. 26 illustrates the penetrating ability of 1.5% IEIK13 compared to 1.0% RADA16 with 0.00585% Indigo Carmine. 1.0% RADA16 was decidedly better in regards to lung volume reduction, particularly in terms of penetration into lung tissue.

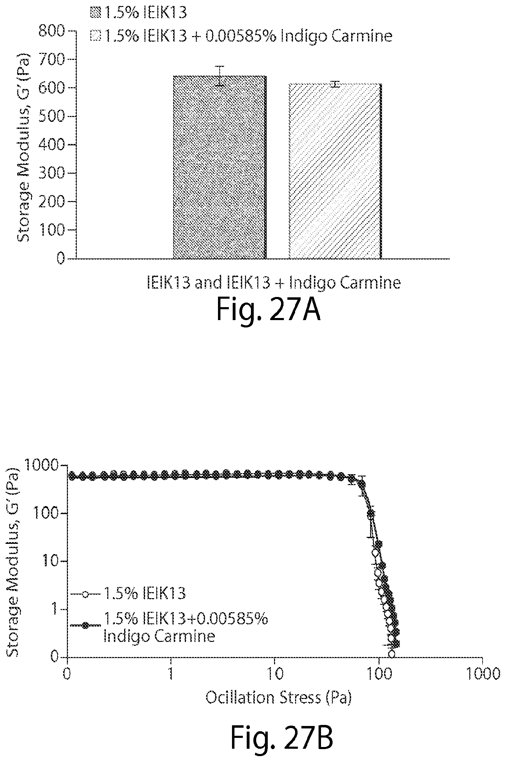

Visualization of hydrogel spreading throughout the lumen of the lungs was necessary for determination of feasibility. Indigo Carmine was chosen as a common contrast reagent. Since Indigo Carmine is a salt-based solution it was possible that the mechanical properties of the gels would be altered. To test this, the mechanical properties of 1.5% IEIK13 was compared to 1.5% IEIK13 with 0.00585% Indigo Carmine 24 hours post reconstitution. IEIK13 was optimal since it has a higher sensitivity to cations/anions than RADA16. The comparison is apparent from FIG. 27 which shows that there is no significant difference when Indigo Carmine was mixed with IEIK13. It was concluded that 0.00585% Indigo Carmine did not significantly affect the mechanical properties of IEIK13 or RADA16. FIGS. 27A and27B provide a rheological representation of the mechanical properties of IEIK13 and IEIK13 + 0.00585% Indigo Carmine.

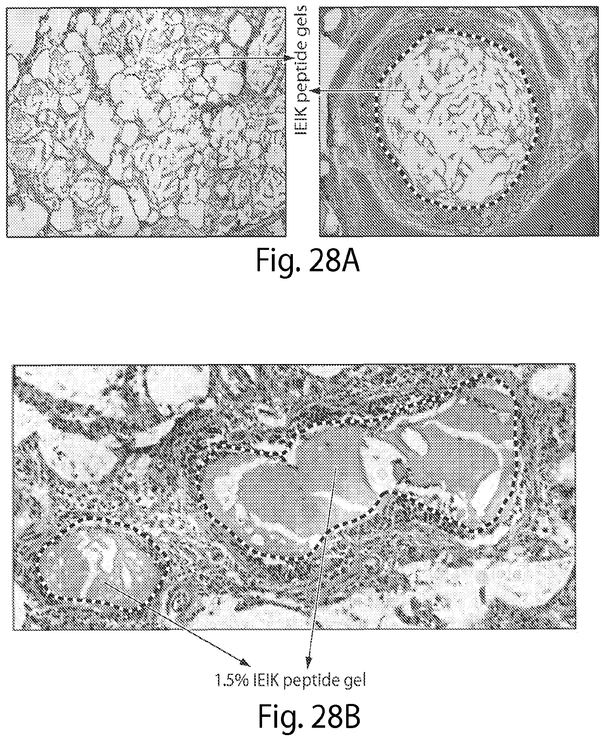

Tissue samples were fixed in paraformaldehyde and rinsed with ethanol. Histology was conducted at Beth Israel Deaconess Medical Center. IEIK13 and RADA16 filled both major and minor lumens of the lungs. As seen in FIGS. 28A-28B, the IEIK13 filled both the major and minor lumens of the bronchi. FIG. 28A presents the IEIK13 gel observed within the major lumens. FIG. 28B presents the IEIK13 gel observed within the minor lumens. Due to H&E processing, peptide gels break/fragment. Optical imaging has shown that these gels remain as bulk structures.

### Conclusion

The use of injectable, self-assembling peptide hydrogel systems for lung volume reduction is a feasible treatment for severe chronic obstructive pulmonary diseases (COPD) patients. As determined from the experiments noted above, the hydrogels of 1.5% IEIK13 and 1.0% RADA16, preferably, can be used to reduce lung volume used for oxygen exchange.

Gross visual inspection revealed that the major lumens were filled 80-100%, and small lumens may exhibit the same degree of penetration.

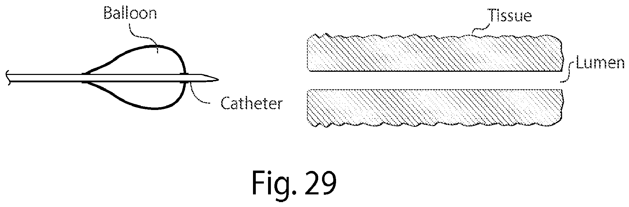

FIG. 29 presents a schematic of an endotracheal tube combination that may be used to prevent backflow of the hydrogel out of the lumen during injection in accordance with the above protocols. A catheter-like balloon attached to an endotracheal tube may prevent backflow of hydrogel out of the lumen and allow for greater penetration of the hydrogel into additional minor lumen.

## Claims

1. A solution comprising a self-assembling peptide comprising between about 7 amino acids and 32 amino acids
wherein at least a portion of the peptide is amphiphilic, such that the peptide may exhibit a beta-sheet structure in aqueous solution in a presence of physiological conditions at a bronchial obstruction site;
the solution being for use in an effective amount and in an effective concentration to administer to a target area to form a hydrogel barrier under physiological conditions of the target area to reduce lung volume and fill at least a portion of the lung of the subject with the solution;
wherein the solution is introduced through a delivery device following the step of introducing a delivery device to a target area of the lung of the subject and positioning an end of the delivery device in the target area in which the bronchial obstruction is desired;
and wherein the delivery device is removed from the target area.

2. The solution of claim 1, wherein any one or more of the following applies,
a) identifying a lobe or a portion of a lobe as the target area;
b) wherein positioning an end of the delivery device in the target area comprises positioning an endotracheal tube in the target area,
optionally wherein positioning an end of the delivery device in the target area comprises inserting the delivery device through a trachea and primary bronchus into a distal end of the target area;
c) retracting the delivery device a predetermined distance after administration of a first volume of solution,
optionally, repeating the administration and retracting until a volume of the target is filled;
d) preventing a backflow of the solution using an endotracheal tube and a catheter.

3. The solution of claim 1, wherein the hydrogel barrier penetrates both a major lumen and a minor lumen of the bronchi and/or the hydrogel barrier is formed in less than about five minutes.

4. The solution of claim 1, wherein any one or more of the following applies,
a) preparing the solution comprising the self-assembling peptide.
b) further comprising adjusting the pH of the solution;
c) wherein the effective amount is approximately 1 mL per 1 cm2 of target area, or
the amount effective to allow treatment of the bronchial obstruction comprises a volume in a range of about 0.1 mL to about 10 mL.

5. The solution of claim 1, wherein the self-assembling peptide is selected from the group consisting of (RADA)4 (SEQ ID NO: 1), (IEIK)3I (SEQ ID NO: 2), and (KLDL)3 (SEQ ID NO: 3).

6. The solution of claim 5, wherein the concentration effective to allow treatment of the pulmonary leakage comprises a self-assembling peptide concentration in a range of about 0.1 weight per volume (w/v) percent to about 3 w/v percent.

7. The solution of claim 4, wherein any one or more of the following applies,
a) preparing the solution comprising the self-assembling peptide comprises adding the self-assembling peptide to a salt solution, or
b) preparing the solution comprising the self-assembling peptide comprises:
adding water to a peptide powder of the self-assembling peptide to provide an aqueous peptide solution;
adding a salt solution to the aqueous peptide solution; and
mixing the salt solution and the aqueous peptide solution;
wherein optionally the salt solution comprises at least one cation selected from the group consisting of ammonium, iron, magnesium, potassium, pyrimidium, quaternary ammonium, sodium, and calcium and/or
at least one anion selected from the group consisting of chloride, sulfate, acetate, carbonate, citrate, cyanide, fluoride, nitrate, nitrite, and phosphate.

8. The solution of claim 4, wherein any one or more of the following applies,
a) the solution comprising the self-assembling peptide comprises (RADA)4 (SEQ ID NO: 1) at a concentration of about 0.5 weight per volume (w/v) percent,
wherein optionally the solution comprising the self-assembling peptide comprises a concentration of calcium chloride of about 0.125 M,
wherein optionally the solution comprising the self-assembling peptide has a storage modulus of about 25 Pa;
b) the solution comprising the self-assembling peptide has a concentration of salt of between about 0.005 M and about 1 M;
c) further comprising a solution comprising sodium chloride, potassium chloride, calcium chloride, and sodium bicarbonate; and
d) the solution has a pH of about 2.5 to about 4.0,
wherein optionally the solution has a pH of about 3.5, and the self-assembling peptide is one of (RADA)4 (SEQ ID NO: 1) and (KLDL)3 (SEQ ID NO: 3), or
the solution has a pH of about 3.7, and the self-assembling peptide is (IEIK)3I (SEQ ID NO: 2).

9. The solution of claim 7, wherein preparing the solution comprising the self-assembling peptide comprises one of adding the self-assembling peptide to a buffer and adding a buffer to the solution, wherein any one or more of the following applies,
a) the buffer comprises at least two salts,
wherein the buffer is at a pH of 7.2 or 7.4;
b) the buffer is an alkali buffer;
c) the solution is buffered with about 0.15 M of at least one of sodium chloride, potassium chloride, magnesium chloride, and calcium chloride,
wherein the buffer comprises between about 0.6 M and about 1.2 M of a salt, and the self-assembling peptide is (RADA)4 (SEQ ID NO: 1), or
the buffer comprises between about 0.02 M and about 0.04 M of a salt, and the self-assembling peptide is (IEIK)3I (SEQ ID NO: 2), or
the buffer comprises between about 0.1 M and about 0.4 M of a salt and the self-assembling peptide is (KLDL)3 (SEQ ID NO: 3).

10. The solution of claim 1, wherein any one or more of the following applies,
a) the solution further comprises at least one biologically active agent;
b) the solution is substantially free of cells and/or drugs; and
c) further comprising a solution comprising a contrasting agent, wherein optionally the contrast agent comprises sulfate ions and sodium ions.

11. A kit for providing a bronchial obstruction in a subject, comprising:
a self-assembling peptide comprising between about 7 amino acids and about 32 amino acids,
wherein at least a portion of the peptide is amphiphilic, such that the peptide may exhibit a beta-sheet structure in aqueous solution in a presence of physiological conditions at a bronchial obstruction site,
in an effective amount to form a hydrogel barrier under physiological conditions to reduce lung volume and fill at least a portion of the lung of the subject with the solution; and
instructions for administering the self-assembling peptide to a target area of the lung of the subject.

12. The kit of claim 11, wherein the self-assembling peptide is provided as one of a solution comprising a self-assembling peptide and a powder to be prepared as a solution comprising a self-assembling peptide.

13. The kit of claim 12, wherein any one or more of the following applies,
a) the concentration effective to reduce lung volume and fill at least a portion of the lung of the subject with the solution comprises a concentration of self-assembling peptide in a range of about 0.1 weight per volume (w/v) percent to about 3 w/v percent;
b) the kit further comprising a delivery device to introduce the self-assembling peptide to a target area of the lung, wherein optionally the delivery device comprises an endotracheal tube comprising a catheter-like balloon attached to the endotracheal tube;
c) wherein the self-assembling peptide is selected from the group consisting of (RADA)4 (SEQ ID NO: 1), (IEIK)3I (SEQ ID NO: 2), and (KLDL)3 (SEQ ID NO: 3);
d) the kit further comprising a salt solution wherein optionally,
the salt solution comprises at least one cation selected from the group consisting of ammonium, iron, magnesium, potassium, pyrimidium, quaternary ammonium, sodium, and calcium; and/or
at least one anion selected from the group consisting of chloride, sulfate, acetate, carbonate, citrate, cyanide, fluoride, nitrate, nitrite, and phosphate
wherein optionally the solution comprising the self-assembling peptide comprises a salt concentration of between about 0.005 M and about 0.500 M,
wherein optionally the solution comprising the self-assembling peptide has a storage modulus of between about 25 Pa and about 600 Pa, and/or
the solution comprising the self-assembling peptide has a pH of about 2.5 to about 4.0,
wherein optionally the solution comprising the self-assembling peptide has a pH of about 3.5, and the self-assembling peptide is one of (RADA)4 (SEQ ID NO: 1) and (KLDL)3 (SEQ ID NO: 3), or
the solution comprising the self-assembling peptide has a pH of about 3.7, and the self-assembling peptide is (IEIK)3I (SEQ ID NO: 2).

14. The kit of claim 12, wherein one of the kit or the solution comprising a self-assembling peptide comprises a buffer, wherein optionally anyone or more of the following applies,
a) the buffer comprises at least two salts;
b) the buffer is at a pH of 7.2 or 7.4;
c) the buffer is an alkali buffer;
d) the solution is buffered with about 0.15 M of at least one of sodium chloride, potassium chloride, magnesium chloride, and calcium chloride;
e) the buffer comprises between about 0.6 M and about 1.2 M of a salt, and the self-assembling peptide is (RADA)4 (SEQ ID NO: 1);
f) the buffer comprises between about 0.02 M and about 0.04 M of a salt, and the self-assembling peptide is (IEIK)3I (SEQ ID NO: 2);
g) the buffer comprises between about 0.1 M and about 0.4 M of a salt and the self-assembling peptide is (KLDL)3 (SEQ ID NO: 3).

15. The kit of claim 12, wherein any one or more of the following applies,
a) the kit further comprising at least one biologically active agent;
b) the solution is substantially free of cells and drugs;
c) the kit further comprising a sucrose solution; and
d) the kit further comprising a solution comprising a contrast agent,
wherein optionally the contrast agent comprises sulfate ions and sodium ions.

## Patentansprüche

1. Lösung, umfassend ein selbstanordnendes Peptid, das zwischen etwa 7 Aminosäuren und 32 Aminosäuren umfasst,
wobei wenigstens ein Teil des Peptids amphiphil ist, so dass das Peptid eine Beta-Faltblatt-Struktur in wässriger Lösung in Gegenwart physiologischer Bedingungen an einer Bronchialverschlussstelle zeigen kann;
wobei die Lösung zur Verwendung in einer wirksamen Menge und in einer wirksamen Konzentration zur Verabreichung an einen Zielbereich unter Bildung einer Hydrogelschranke unter physiologischen Bedingungen des Zielbereichs dient, so dass das Lungenvolumen verringert wird und wenigstens ein Teil der Lunge des Individuums mit der Lösung gefüllt wird;
wobei die Lösung über eine Zuführungsvorrichtung nach dem Schritt des Einführens einer Zuführungsvorrichtung an einen Zielbereich der Lunge des Individuums und Positionierens eines Endes der Zuführungsvorrichtung im Zielbereich, in dem der Bronchialverschluss gewünscht ist, eingeführt wird;
und wobei die Zuführungsvorrichtung aus dem Zielbereich entfernt wird.

2. Lösung nach Anspruch 1, wobei einer oder mehrere der folgenden Punkte zutreffen,
a) Identifizieren eines Lappens oder eines Teils eines Lappens als Zielbereich;
b) wobei Positionieren eines Endes der Zuführungsvorrichtung im Zielbereich Positionieren eines Endotrachealtubus im Zielbereich umfasst, gegebenenfalls wobei Positionieren eines Endes der Zuführungsvorrichtung im Zielbereich Einführen der Zuführungsvorrichtung über eine Trachea bzw. einen primären Bronchus in ein distales Ende des Zielbereichs umfasst;
c) Zurückziehen der Zuführungsvorrichtung um eine vorbestimmte Strecke nach Verabreichung eines ersten Volumens an Lösung,
gegebenenfalls Wiederholen der Verabreichung und des Zurückziehens, bis ein Volumen des Ziels gefüllt ist;
d) Verhindern eines Rückflusses der Lösung unter Verwendung eines Endotrachealtubus und eines Katheters.

3. Lösung nach Anspruch 1, wobei die Hydrogelschranke sowohl ein großes Lumen als auch ein kleines Lumen der Bronchi durchdringt und/oder die Hydrogelschranke in weniger als etwa fünf Minuten ausgebildet wird.

4. Lösung nach Anspruch 1, wobei einer oder mehrere der folgenden Punkte zutreffen,
a) Herstellen der Lösung, die das selbstanordnende Peptid umfasst,
b) ferner umfassend Einstellen des pH der Lösung;
c) wobei die wirksame Menge bei ungefähr 1 ml pro 1 cm² Zielbereich liegt oder
die zum Gestatten einer Behandlung des Bronchialverschlusses wirksame Menge ein Volumen in einem Bereich von etwa 0,1 ml bis etwa 10 ml umfasst.

5. Lösung nach Anspruch 1, wobei das selbstanordnende Peptid aus der Gruppe bestehend aus (RADA)4 (SEQ ID NO: 1), (IEIK) 3I (SEQ ID NO: 2) und (KLDL) 3 (SEQ ID NO: 3) ausgewählt ist.

6. Lösung nach Anspruch 5, wobei die zum Gestatten einer Behandlung der Lungenleckage wirksame Konzentration eine Konzentration des selbstanordnenden Peptids in einem Bereich von etwa 0,1-w/v(Gewicht pro Volumen)-Prozent bis etwa 3-w/v-Prozent umfasst.

7. Lösung nach Anspruch 4, wobei einer oder mehrere der folgenden Punkte zutreffen,
a) Herstellen der das selbstanordnende Peptid umfassenden Lösung umfasst Zugeben des selbstanordnenden Peptids zu einer Salzlösung oder
b) Herstellen der das selbstanordnende Peptid umfassenden Lösung umfasst:
Versetzen eines Peptidpulvers des selbstanordnenden Peptids mit Wasser, so dass eine wässrige Peptidlösung bereitgestellt wird;
Versetzen der wässrigen Peptidlösung mit einer Salzlösung; und
Mischen der Salzlösung und der wässrigen Peptidlösung;
wobei gegebenenfalls die Salzlösung wenigstens ein Kation, das aus der Gruppe bestehend aus Ammonium, Eisen, Magnesium, Kalium, Pyrimidium, quartärem Ammonium, Natrium, und Calcium ausgewählt ist, und/oder
wenigstens ein Anion, das aus der Gruppe bestehend aus Chlorid, Sulfat, Acetat, Carbonat, Citrat, Cyanid, Fluorid, Nitrat, Nitrit und Phosphat ausgewählt ist, umfasst.

8. Lösung nach Anspruch 4, wobei einer oder mehrere der folgenden Punkte zutreffen,
a) die das selbstanordnende Peptid umfassende Lösung umfasst (RADA)4 (SEQ ID NO: 1) in einer Konzentration von etwa 0,5-w/v(Gewicht pro Volumen)-Prozent,
wobei gegebenenfalls die das selbstanordnende Peptid umfassende Lösung eine Calciumchloridkonzentration von etwa 0,125 M umfasst,
wobei gegebenenfalls die das selbstanordnende Peptid umfassende Lösung einen Speichermodulus von etwa 25 Pa aufweist;
b) die das selbstanordnende Peptid umfassende Lösung weist eine Salzkonzentration zwischen etwa 0,005 M und etwa 1 M auf;
c) ferner umfassend eine Lösung, die Natriumchlorid, Kaliumchlorid, Calciumchlorid und Natriumhydrogencarbonat umfasst; und
d) die Lösung weist einen pH-Wert von etwa 2,5 bis etwa 4,0 auf,
wobei gegebenenfalls die Lösung einen pH-Wert von etwa 3,5 aufweist und es sich bei dem selbstanordnenden Peptid um entweder (RADA)4 (SEQ ID NO: 1) oder (KLDL)3 (SEQ ID NO: 3) handelt, oder die Lösung einen pH-Wert von etwa 3,7 aufweist und es sich bei dem selbstanordnenden Peptid um (IEIK)3I (SEQ ID NO: 2) handelt.

9. Lösung nach Anspruch 7, wobei Herstellen der das selbstanordnende Peptid umfassenden Lösung entweder Zugeben des selbstanordnenden Peptids zu einem Puffer oder Zugeben eines Puffers zur Lösung umfasst, wobei einer oder mehrere der folgenden Punkte zutreffen,
a) der Puffer umfasst wenigstens zwei Salze, wobei der Puffer mit einem pH-Wert von 7,2 oder 7,4 vorliegt;
b) bei dem Puffer handelt es sich um einen Alkalipuffer;
c) die Lösung ist mit etwa 0,15 M von wenigstens einem von Natriumchlorid, Kaliumchlorid, Magnesiumchlorid und Calciumchlorid gepuffert,
wobei der Puffer zwischen etwa 0,6 M und etwa 1,2 M eines Salzes umfasst und es sich bei dem selbstanordnenden Peptid um (RADA)4 (SEQ ID NO: 1) handelt oder
der Puffer zwischen etwa 0,02 M und etwa 0,04 M eines Salzes umfasst und es sich bei dem selbstanordnenden Peptid um (IEIK)3I (SEQ ID NO: 2) handelt oder
der Puffer zwischen etwa 0,1 M und etwa 0,4 M eines Salzes umfasst und es sich bei dem selbstanordnenden Peptid um (KLDL)3 (SEQ ID NO: 3) handelt.

10. Lösung nach Anspruch 1, wobei einer oder mehrere der folgenden Punkte zutreffen,
a) die Lösung umfasst ferner wenigstens einen biologischen Wirkstoff;
b) die Lösung ist weitgehend frei von Zellen und/oder Arzneistoffen; und
c) ferner umfassend eine Lösung, die ein Kontrastmittel umfasst, wobei gegebenenfalls das Kontrastmittel Sulfationen und Natriumionen umfasst.

11. Kit zum Bereitstellen eines Bronchialverschlusses bei einem Individuum, umfassend:
ein selbstanordnendes Peptid, das zwischen etwa 7 Aminosäuren und etwa 32 Aminosäuren umfasst,
wobei wenigstens ein Teil des Peptids amphiphil ist, so dass das Peptid eine Beta-Faltblatt-Struktur in wässriger Lösung in Gegenwart physiologischer Bedingungen an einer Bronchialverschlussstelle zeigen kann;
in einer wirksamen Menge zur Bildung einer Hydrogelschranke unter physiologischen Bedingungen, so dass das Lungenvolumen verringert wird und wenigstens ein Teil der Lunge des Individuums mit der Lösung gefüllt wird; und
Anweisungen zum Verabreichen des selbstanordnenden Peptids an einen Zielbereich der Lunge des Individuums.

12. Kit nach Anspruch 11, wobei das selbstanordnende Peptid als entweder eine ein selbstanordnendes Peptid umfassende Lösung oder ein als eine ein selbstanordnendes Peptid umfassende Lösung zuzubereitendes Pulver bereitgestellt wird.

13. Kit nach Anspruch 12, wobei einer oder mehrere der folgenden Punkte zutreffen,
a) die zum Verringern des Lungenvolumens und Füllen wenigstens eines Teils der Lunge des Individuums mit der Lösung wirksame Konzentration umfasst eine Konzentration von selbstanordnendem Peptid in einem Bereich von etwa 0,1-w/v(Gewicht pro Volumen)-Prozent bis etwa 3-w/v-Prozent;
b) wobei das Kit ferner eine Zuführungsvorrichtung zum Einführen des selbstanordnenden Peptids an einen Zielbereich der Lunge umfasst, wobei gegebenenfalls die Zuführungsvorrichtung einen Endotrachealtubus umfasst, der einen am Endotrachealtubus angebrachten katheterähnlichen Ballon umfasst;
c) wobei das selbstanordnende Peptid aus der Gruppe bestehend aus (RADA)4 (SEQ ID NO: 1), (IEIK) 3I (SEQ ID NO: 2) und (KLDL) 3 (SEQ ID NO: 3) ausgewählt ist;
d) wobei das Kit ferner eine Salzlösung umfasst, wobei gegebenenfalls
die Salzlösung wenigstens ein Kation, das aus der Gruppe bestehend aus Ammonium, Eisen, Magnesium, Kalium, Pyrimidium, quartärem Ammonium, Natrium, und Calcium ausgewählt ist, und/oder
wenigstens ein Anion, das aus der Gruppe bestehend aus Chlorid, Sulfat, Acetat, Carbonat, Citrat, Cyanid, Fluorid, Nitrat, Nitrit und Phosphat ausgewählt ist, umfasst,
wobei gegebenenfalls die das selbstanordnende Peptid umfassende Lösung eine Salzkonzentration zwischen etwa 0,005 M und etwa 0,500 M umfasst,
wobei gegebenenfalls die das selbstanordnende Peptid umfassende Lösung einen Speichermodulus zwischen etwa 25 Pa und etwa 600 Pa aufweist und/oder
die das selbstanordnende Peptid umfassende Lösung einen pH-Wert von etwa 2,5 bis etwa 4,0 aufweist,
wobei gegebenenfalls die das selbstanordnende Peptid umfassende Lösung einen pH-Wert von etwa 3,5 aufweist und es sich bei dem selbstanordnenden Peptid um entweder (RADA)4 (SEQ ID NO: 1) oder (KLDL)3 (SEQ ID NO: 3) handelt, oder
die das selbstanordnende Peptid umfassende Lösung einen pH-Wert von etwa 3,7 aufweist und es sich bei dem selbstanordnenden Peptid um (IEIK)3I (SEQ ID NO: 2) handelt.

14. Kit nach Anspruch 12, wobei entweder das Kit oder die ein selbstanordnendes Peptid umfassende Lösung einen Puffer umfasst, wobei gegebenenfalls einer oder mehrere der folgenden Punkte zutreffen,
a) der Puffer umfasst wenigstens zwei Salze;
b) der Puffer liegt mit einem pH-Wert von 7,2 oder 7,4 vor;
c) bei dem Puffer handelt es sich um einen Alkalipuffer;
d) die Lösung ist mit etwa 0,15 M von wenigstens einem von Natriumchlorid, Kaliumchlorid, Magnesiumchlorid und Calciumchlorid gepuffert;
e) der Puffer umfasst zwischen etwa 0,6 M und etwa 1,2 M eines Salzes, und bei dem selbstanordnenden Peptid handelt es sich um (RADA)4 (SEQ ID NO: 1);
f) der Puffer umfasst zwischen etwa 0,02 M und etwa 0,04 M eines Salzes, und bei dem selbstanordnenden Peptid handelt es sich um (IEIK)3I (SEQ ID NO: 2);
g) der Puffer umfasst zwischen etwa 0,1 M und etwa 0,4 M eines Salzes, und bei dem selbstanordnenden Peptid handelt es sich um (KLDL)3 (SEQ ID NO: 3).

15. Kit nach Anspruch 12, wobei einer oder mehrere der folgenden Punkte zutreffen,
a) wobei das Kit ferner wenigstens einen biologischen Wirkstoff umfasst;
b) die Lösung ist weitgehend frei von Zellen und/oder Arzneistoffen;
c) wobei das Kit ferner eine Saccharoselösung umfasst; und
d) wobei das Kit ferner eine Lösung umfasst, die ein Kontrastmittel umfasst,
wobei gegebenenfalls das Kontrastmittel Sulfationen und Natriumionen umfasst.

## Revendications

1. Solution comprenant un peptide s'auto-assemblant, qui comprend entre 7 acides aminés et 32 acides aminés environ
dans laquelle au moins une partie du peptide est amphiphile, de sorte que le peptide puisse présenter une structure en feuillet bêta en solution aqueuse en présence de conditions physiologiques au niveau d'un site d'obstruction bronchique ;
la solution étant destinée à être utilisée en une quantité efficace et à une concentration efficace pour une administration à une surface cible pour former une barrière d'hydrogel, dans des conditions physiologiques de la surface cible, afin de réduire le volume pulmonaire et remplir avec la solution au moins une partie du poumon du sujet ;
la solution étant introduite au moyen d'un dispositif de délivrance après les étapes d'introduction d'un dispositif de délivrance au niveau d'une surface cible du poumon du sujet et de positionnement d'une extrémité du dispositif de délivrance dans la surface cible dans laquelle l'obstruction bronchique est souhaitée ;
et dans laquelle le dispositif de délivrance est ôté de la surface cible.

2. Solution de la revendication 1, dans laquelle un ou plusieurs des élément(s) suivant(s) s'applique/s'appliquent,
a) identifier un lobe ou une partie de lobe comme étant la surface cible ;
b) dans laquelle un positionnement d'une extrémité du dispositif de délivrance dans la surface cible comprend un positionnement d'un tube endotrachéal dans la surface cible,
dans laquelle, en option, un positionnement d'une extrémité du dispositif de délivrance dans la surface cible comprend une insertion du dispositif de délivrance, à travers une trachée et une bronche primaire, dans une extrémité distale de la surface cible ;
c) rétracter le dispositif de délivrance par une distance prédéterminée après une administration d'un premier volume de solution,
en option, répéter l'administration et la rétractation jusqu'à ce qu'un volume de la cible soit rempli ;
d) empêcher un reflux de la solution en utilisant un tube endotrachéal et un cathéter.

3. Solution de la revendication 1, dans laquelle la barrière d'hydrogel pénètre à la fois dans un lumen principal et un lumen secondaire des bronches et/ou la barrière d'hydrogel est formée en moins de cinq minutes environ.

4. Solution de la revendication 1, dans laquelle un ou plusieurs des élément(s) suivant(s) s'applique/s'appliquent,
a) préparer la solution comprenant le peptide s'auto-assemblant.
b) comprenant en plus l'ajustement du pH de la solution ;
c) dans laquelle la quantité efficace est approximativement de 1 mL par cm² de surface cible ou la quantité efficace pour permettre un traitement de l'obstruction bronchique comprend un volume dans une gamme allant de 0,1 mL environ à 10 mL environ.

5. Solution de la revendication 1, dans laquelle le peptide s'auto-assemblant est choisi dans le groupe constitué par les (RADA)4 (SEQ ID n° : 1), (IEIK)31 (SEQ ID n° : 2) et (KLDL)3 (SEQ ID n° : 3).

6. Solution de la revendication 5, dans laquelle la concentration efficace pour permettre un traitement de la fuite pulmonaire comprend une concentration en peptide s'auto-assemblant comprise dans une gamme allant de 0,1 pour cent en poids par volume (p/v) environ à 3 pour cent p/v environ.

7. Solution de la revendication 4, dans laquelle un ou plusieurs des élément(s) suivant(s) s'applique/s'appliquent,
a) la préparation de la solution comprenant le peptide s'auto-assemblant comprend un ajout du peptide s'auto-assemblant à une solution saline ou
b) la préparation de la solution comprenant le peptide s'auto-assemblant comprend :
un ajout d'eau à une poudre de peptide du peptide s'auto-assemblant pour fournir une solution peptidique aqueuse ;
un ajout d'une solution saline à la solution peptidique aqueuse ; et
le mélange de la solution saline et la solution peptidique aqueuse ;
dans laquelle, en option, la solution saline comprend au moins un cation choisi dans le groupe constitué par les ammonium, fer, magnésium, potassium, pyrimidium, ammonium quaternaire, sodium, et calcium, et/ou
au moins un anion choisi dans le groupe constitué par les chlorure, sulfate, acétate, carbonate, citrate, cyanure, fluorure, nitrate, nitrite et phosphate.

8. Solution de la revendication 4, dans laquelle un ou plusieurs des élément(s) suivant(s) s'applique/s'appliquent,
a) la solution comprenant le peptide s'auto-assemblant comprend (RADA)4 (SEQ ID n° : 1) en une concentration de 0,5 pour cent en poids par volume (p/v) environ,
la solution, qui comprend le peptide s'auto-assemblant, comprenant une concentration en chlorure de calcium de 0,125 M environ,
en option, la solution qui comprend le peptide s'auto-assemblant ayant un module de stockage de 25 Pa environ ;
b) la solution comprenant le peptide s'auto-assemblant a une concentration en sel comprise entre 0,005 M environ et 1 M environ ;
c) comprenant en outre une solution qui comprend des chlorure de sodium, chlorure de potassium, chlorure de calcium et bicarbonate de sodium ; et
d) la solution a un pH allant de 2,5 environ à 4,0 environ,
en option, la solution ayant un pH de 3,5 environ et le peptide s'auto-assemblant en est un parmi les (RADA)4 (SEQ ID n° : 1) et (KLDL)3 (SEQ ID n° : 3) ou
la solution ayant un pH de 3,7 environ et le peptide s'auto-assemblant est (IEIK)31 (SEQ ID n° : 2).

9. Solution de la revendication 7, dans laquelle la préparation de la solution comprenant le peptide s'auto-assemblant comprend un élément parmi un ajout du peptide s'auto-assemblant à un tampon et un ajout d'un tampon à la solution, dans laquelle un ou plusieurs des élément(s) suivant(s) s'applique/s'appliquent,
a) le tampon comprend au moins deux sels,
dans laquelle le tampon est à un pH de 7,2 ou 7,4 ;
b) le tampon est un tampon alcalin ;
c) la solution est tamponnée avec 0,15 M environ d'au moins un élément parmi les chlorure de sodium, chlorure de potassium, chlorure de magnésium et chlorure de calcium,
dans laquelle le tampon comprend entre 0,6 M environ et 1,2 M environ d'un sel et le peptide s'auto-assemblant est (RADA)4 (SEQ ID n° : 1) ou
le tampon comprend entre 0,02 M environ et 0,04 M environ d'un sel et le peptide s'auto-assemblant est (IEIK)31 (SEQ ID n° : 2) ou
le tampon comprend entre 0,1 M environ et 0,4 M environ d'un sel et le peptide s'auto-assemblant est (KLDL)3 (SEQ ID n° : 3).

10. Solution de la revendication 1, dans laquelle un ou plusieurs des élément(s) suivant(s) s'applique/s'appliquent,
a) la solution comprend en outre au moins un agent biologiquement actif ;
b) la solution est substantiellement dépourvue de cellules et/ou de substances médicamenteuses ; et
c) comprenant en outre une solution qui comprend un agent de contraste, dans laquelle, en option, l'agent de contraste comprend des ions sulfate et ions sodium.

11. Trousse destinée à fournir une obstruction bronchique chez un sujet, comprenant :
un peptide s'auto-assemblant, qui comprend entre 7 acides aminés environ et 32 acides aminés environ,
dans laquelle au moins une partie du peptide est amphiphile, de sorte que le peptide puisse présenter une structure en feuillet bêta en solution aqueuse en présence de conditions physiologiques au niveau d'un site d'obstruction bronchique,
en une quantité efficace pour former une barrière d'hydrogel dans des conditions physiologiques pour réduire le volume pulmonaire et remplir avec la solution au moins une partie du poumon du sujet ; et
des instructions pour administrer le peptide s'auto-assemblant à une surface cible du poumon du sujet.

12. Trousse de la revendication 11, dans laquelle le peptide s'auto-assemblant est fourni comme étant un élément d'une solution comprenant un peptide s'auto-assemblant et une poudre pour préparation d'une solution qui comprend un peptide s'auto-assemblant.

13. Trousse de la revendication 12, dans laquelle un ou plusieurs des élément(s) suivant(s) s'applique/s'appliquent,
a) la concentration efficace pour réduire le volume pulmonaire et remplir au moins une partie du poumon du sujet avec la solution comprend une concentration en peptide s'auto-assemblant comprise dans une gamme allant de 0,1 pour cent en poids par volume (p/v) environ à 3 pour cent p/v environ ;
b) la trousse comprenant en outre un dispositif de délivrance destiné à introduire le peptide s'auto-assemblant dans une surface cible du poumon, dans laquelle, en option, le dispositif de délivrance comprend un tube endotrachéal qui comprend un ballon de type cathéter fixé au tube endotrachéal ;
c) dans laquelle le peptide s'auto-assemblant est choisi dans le groupe constitué par les (RADA)4 (SEQ ID n° : 1), (IEIK) 31 (SEQ ID n° : 2) et (KLDL) 3 (SEQ ID n° : 3) ;
d) la trousse comprenant en outre une solution saline dans laquelle, en option,
la solution saline comprend au moins un cation choisi dans le groupe constitué par les ammonium, fer, magnésium, potassium, pyrimidium, ammonium quaternaire, sodium, et calcium ; et/ou
au moins un anion choisi dans le groupe constitué par les chlorure, sulfate, acétate, carbonate, citrate, cyanure, fluorure, nitrate, nitrite et phosphate,
dans laquelle, en option, la solution comprenant le peptide s'auto-assemblant comprend une concentration en sel comprise entre 0,005 M environ et 0,500 M environ ; dans laquelle, en option, la solution comprenant le peptide s'auto-assemblant a un module de stockage compris entre 25 Pa environ et 600 Pa environ et/ou
la solution comprenant le peptide s'auto-assemblant a un pH allant de 2,5 environ à 4,0 environ,
dans laquelle, en option, la solution comprenant le peptide s'auto-assemblant a un pH de 3,5 environ et le peptide s'auto-assemblant en est un parmi les (RADA)4 (SEQ ID n° : 1) et (KLDL)3 (SEQ ID n° : 3) ou
la solution comprenant le peptide s'auto-assemblant a un pH de 3,7 environ et le peptide s'auto-assemblant est (IEIK)31 (SEQ ID n° : 2).

14. Trousse de la revendication 12, dans laquelle un élément parmi la trousse ou la solution comprenant un peptide s'auto-assemblant comprend un tampon, en option dans laquelle un ou plusieurs des élément(s) suivant(s) s'applique/s'appliquent,
a) le tampon comprend au moins deux sels ;
b) le tampon est à un pH de 7,2 ou 7,4 ;
c) le tampon est un tampon alcalin ;
d) la solution est tamponnée avec 0,15 M environ d'au moins un élément parmi les chlorure de sodium, chlorure de potassium, chlorure de magnésium et chlorure de calcium ;
e) le tampon comprend entre 0,6 M environ et 1,2 M environ d'un sel et le peptide s'auto-assemblant est (RADA)4 (SEQ ID n° : 1) ;
f) le tampon comprend entre 0,02 M environ et 0,04 M environ d'un sel et le peptide s'auto-assemblant est (IEIK)31 (SEQ ID n° : 2) ;
g) le tampon comprend entre 0,1 M environ et 0,4 M environ d'un sel et le peptide s'auto-assemblant est (KLDL)3 (SEQ ID n° : 3).

15. Trousse de la revendication 12, dans laquelle un ou plusieurs des élément(s) suivant(s) s'applique/s'appliquent,
a) la trousse comprenant en outre au moins un agent biologiquement actif ;
b) la solution est substantiellement dépourvue de cellules et de substances médicamenteuses ;
c) la trousse comprenant en outre une solution de saccharose ; et
d) la trousse comprenant en outre une solution qui comprend un agent de contraste,
dans laquelle, en option, l'agent de contraste comprend des ions sulfate et ions sodium.
